# EUROPEAN PATENT APPLICATION

(11) **EP 4 414 388 A1**
(43) Date of publication of application: **14.08.2024**
(21) Application number: 22889249.3
(22) Date of filing: 01.11.2022
(51) Int. Cl.: C07K 16/28, A61K 39/395, A61P 35/00

(54) **NOVEL ANTI-L1CAM ANTIBODY**

(30) Priority: 03.11.2021 CN 202111296133; 10.01.2022 CN 202210022509
(71) Applicant: Curon Biopharmaceutical (Shanghai) Co., Limited, Shanghai 201309 (CN)
(72) Inventor: WANG, Ji, Shanghai 201309 (CN); DING, Zhilou, Shanghai 201309 (CN); CHEN, Zhihong, Shanghai 201309 (CN); GU, Jinming, Shanghai 201309 (CN); CUI, Dongbing, Shanghai 201309 (CN); YANG, Qiumei, Shanghai 201309 (CN)
(74) Representative: E. Blum & Co. AG
(86) International application number: PCT/CN2022/128854
(87) International publication number: WO 2023/078224

(57) **Abstract**

The invention provides a fully humanized monoclonal antibody which binds to a human L1CAM with a high specificity, an antigen-binding fragment thereof, a bispecific antibody thereof, etc. The antibodies of the present invention can be used in the diagnosis and treatment of cancer.

## Description

The application claims the priority of Chinese application 202111296133.4 filed on November 3, 2021 and Chinese application 202210022509.0 filed on January 10, 2022, the entire contents of which are incorporated herein by reference.

### Technical Field

The invention relates to the field of biotechnology, and in particular relates to fully human monoclonal antibodies, antigen-binding fragments and chimeric antigen receptors specifically binding to human L1CAM, and nucleic acids required for constructing the antibodies, antigen-binding fragments and chimeric antigen receptors , plasmids, host cells, corresponding pharmaceutical compositions and applications thereof.

### Background

L1 cell adhesion molecule (L1 cell adhesion molecule), also known as CD171, is a transmembrane glycoprotein with a molecular weight between 200,000 and 220,000 [Miriam van der Maten et al., Int. J. Mol. Sci ., 2019, 20:4180.]. The expression of L1 cell adhesion molecules in healthy people is mainly limited to the nervous system, with a small amount of expression in kidney tissue and skin [Annette Kunkele et al., Clin. Cancer Res., 2016, 23:466-477.]. L1 cell adhesion molecule has a high expression level in various tumors, and is closely related to the proliferation and drug resistance of various tumor cells.

L1 cell adhesion molecule is a potential tumor therapeutic target, but there is still a need for a therapeutic antibody against L1 cell adhesion molecule.

### Contents of the invention

The inventors of the present invention obtained an anti-L1CAM monoclonal antibody with high specificity, high affinity and high stability by using hybridoma technology through screening.

In a first aspect, the present invention provides a L1CAM binding molecule comprising a fully human immunoglobulin that specifically binds L1CAM.

In another aspect, the present invention relates to nucleic acid molecules encoding L1CAM binding molecules and expression vectors and host cells containing said nucleic acid molecules.

In addition, the present invention also discloses nucleic acids encoding these fully cloned antibodies and antigen-binding fragments. In some embodiments, vectors comprising these nucleic acids, and host cells comprising these vectors are disclosed. In further embodiments, pharmaceutical compositions comprising these fully clonal antibodies, antigen-binding fragments, bispecific antibodies, immunoconjugates, fusion proteins, nucleic acids, and carriers are disclosed. In other embodiments, these fully cloned antibodies, antigen-binding fragments, bispecific antibodies, immunoconjugates, fusion proteins, nucleic acids, and vectors are used to diagnose or treat autoimmune diseases, chronic viral infections, and tumors.

The present invention discloses a novel fully human monoclonal antibody specific to L1CAM. By using this antibody CDR region or part or whole gene, different forms of genetically engineered antibodies can be modified and produced in prokaryotic and eukaryotic cells and any expression system, which can be used for clinical diagnosis or treatment of autoimmune diseases, chronic viral infections and tumors, etc. disease.

The present invention discloses a fully human monoclonal antibody specifically binding to human L1CAM, and the CDR region of the variable region of the light chain and/or the variable region of the heavy chain. In some embodiments, the amino acid sequence of the light chain variable region and/or the amino acid sequence of the heavy chain variable region of the monoclonal antibody or antigen-binding fragment comprises at least one CDR region of the light chain and/or heavy chain variable region . The present invention also discloses antigen-binding fragments of these monoclonal antibodies, bispecific antibodies, chimeric antigen receptor CARs, antibody conjugates, corresponding nucleic acids, plasmids and host cells, corresponding pharmaceutical compositions, and cancer use in diagnosis and treatment.

The present invention provides an antibody or antigen binding fragment thereof specifically binding to L1CAM comprising a light chain variable region comprising light chain complementarity determining regions LCDR1, LCDR2 and LCDR3 and/or a heavy chain variable region comprising heavy chain complementarity determining regions HCDR1, HCDR2 and HCDR3, wherein the antibody or antigen binding fragment thereof can specifically bind to the Ig1 or FN-4 domain of L1-CAM and does not have the same or equivalent combination of CDRs as antibody L1-9. 3 or UJ127.

In one embodiment, wherein the heavy chain variable region comprises HCDR1, HCDR2 and HCDR3 comprising at least 80%, 90% or 100% identity to the HCDR1, HCDR2 and HCDR3 of the heavy chain variable region sequences selected from the group comprising SEQ ID NOs: 43, 45, 47, 49 and 51, and the light chain variable region comprises LCDR1, LCDR2 and LCDR3 comprising at least 80% identity to the LCDR1, LCDR2 and LCDR3 of the light chain variable region sequences selected from the group comprising SEQ ID NOs: 44, 46, 48, 50 and 52.

In one embodiment, wherein comprising a heavy chain variable region and a light chain variable region, wherein the heavy chain variable region and the light chain variable region have a combination of CDRs selected from the group comprising of:
(1) HCDR1, HCDR2 and HCDR3 comprising at least 80% identity to HCDR1, HCDR2 and HCDR3 of the heavy chain variable region sequence of SEQ ID NO: 43, LCDR1, LCDR2 and LCDR3 comprising at least 80% identity to LCDR1, LCDR2 and LCDR3 of the light chain variable region sequence of SEQ ID NO: 44;
(2) HCDR1, HCDR2 and HCDR3 comprising at least 80% identity to HCDR1, HCDR2 and HCDR3 of the heavy chain variable region sequence of SEQ ID NO: 45, LCDR1, LCDR2 and LCDR3 comprising at least 80% identity to LCDR1, LCDR2 and LCDR3 of the light chain variable region sequence of SEQ ID NO: 46;
(3) HCDR1, HCDR2 and HCDR3 comprising at least 80% identity to HCDR1, HCDR2 and HCDR3 of the heavy chain variable region sequence of SEQ ID NO: 47, LCDR1, LCDR2 and LCDR3 comprising at least 80% identity to LCDR1, LCDR2 and LCDR3 of the light chain variable region sequence of SEQ ID NO: 48;
(4) HCDR1, HCDR2 and HCDR3 comprising at least 80% identity to HCDR1, HCDR2 and HCDR3 of the heavy chain variable region sequence of SEQ ID NO: 49, LCDR1, LCDR2 and LCDR3 comprising at least 80% identity to LCDR1, LCDR2 and LCDR3 of the light chain variable region sequence of SEQ ID NO: 50; And
(5) HCDR1, HCDR2 and HCDR3 comprising at least 80% identity to HCDR1, HCDR2 and HCDR3 of the heavy chain variable region sequence of SEQ ID NO: 51, LCDR1, LCDR2 and LCDR3 comprising at least 80% identity to LCDR1, LCDR2 and LCDR3 of the light chain variable region sequence of SEQ ID NO: 52.

In one embodiment, wherein:
(1) the HCDR1 comprises at least 80% identity to a sequence selected from SEQ ID NO: 1, SEQ ID NO: 7, SEQ ID NO: 13, SEQ ID NO: 19, SEQ ID NO: 25, SEQ ID NO: 31, SEQ ID NO: 37;
(2) the HCDR2 comprises at least 80% identity to a sequence selected from SEQ ID NO: 2, SEQ ID NO: 8, SEQ ID NO: 14, SEQ ID NO: 20, SEQ ID NO: 26, SEQ ID NO: 32, SEQ ID NO: 38;
(3) the HCDR3 comprises at least 80% identity to a sequence selected from SEQ ID NO: 3, SEQ ID NO: 9, SEQ ID NO: 15, SEQ ID NO: 21, SEQ ID NO: 27, SEQ ID NO: 33, SEQ ID NO: 39;
(4) the LCDR1 comprises at least 80% identity to a sequence selected from SEQ ID NO: 4, SEQ ID NO: 10, SEQ ID NO: 16, SEQ ID NO: 22, SEQ ID NO: 28, SEQ ID NO: 34, SEQ ID NO: 40;
(5) the LCDR2 comprises at least 80% identity to a sequence selected from SEQ ID NO: 5, SEQ ID NO: 11, SEQ ID NO: 17, SEQ ID NO: 23, SEQ ID NO: 29, SEQ ID NO: 35, SEQ ID NO: 41;
(6) the LCDR3 comprises at least 80% identity to a sequence selected from SEQ ID NO: 6, SEQ ID NO: 12, SEQ ID NO: 18, SEQ ID NO: 24, SEQ ID NO: 30, SEQ ID NO: 36, SEQ ID NO: 42.

In one embodiment, wherein,
(1) the HCDR1 comprises the amino acid sequence set forth in SEQ ID NO: 1;
(2) the HCDR2 comprises the amino acid sequence set forth in SEQ ID NO: 2;
(3) the HCDR3 comprises the amino acid sequence set forth in SEQ ID NO: 3;
(4) the LCDR1 comprises the amino acid sequence set forth in SEQ ID NO: 4;
(5) the LCDR2 comprises the amino acid sequence set forth in SEQ ID NO: 5;
(6) the LCDR3 comprises the amino acid sequence set forth in SEQ ID NO: 6.

In one embodiment, wherein,
(1) the HCDR1 comprises the amino acid sequence set forth in SEQ ID NO: 7;
(2) the HCDR2 comprises the amino acid sequence set forth in SEQ ID NO: 8;
(3) the HCDR3 comprises the amino acid sequence set forth in SEQ ID NO: 9;
(4) the LCDR1 comprises the amino acid sequence set forth in SEQ ID NO: 10;
(5) the LCDR2 comprises the amino acid sequence set forth in SEQ ID NO: 11;
(6) the LCDR3 comprises the amino acid sequence set forth in SEQ ID NO: 12.

In one embodiment, wherein,
(1) the HCDR1 comprises the amino acid sequence set forth in SEQ ID NO: 13;
(2) the HCDR2 comprises the amino acid sequence set forth in SEQ ID NO: 14;
(3) the HCDR3 comprises the amino acid sequence set forth in SEQ ID NO: 15;
(4) the LCDR1 comprises the amino acid sequence set forth in SEQ ID NO: 16;
(5) the LCDR2 comprises the amino acid sequence set forth in SEQ ID NO: 17;
(6) the LCDR3 comprises the amino acid sequence set forth in SEQ ID NO: 18

In one embodiment, wherein,
(1) the HCDR1 comprises the amino acid sequence set forth in SEQ ID NO: 19;
(2) the HCDR2 comprises the amino acid sequence set forth in SEQ ID NO: 20;
(3) the HCDR3 comprises an amino acid sequence as set forth in SEQ ID NO: 21;
(4) the LCDR1 comprises the amino acid sequence set forth in SEQ ID NO: 22;
(5) the LCDR2 comprises the amino acid sequence set forth in SEQ ID NO: 23;
(6) the LCDR3 comprises the amino acid sequence set forth in SEQ ID NO: 24

In one embodiment, wherein,
(1) the HCDR1 comprises the amino acid sequence set forth in SEQ ID NO: 25;
(2) the HCDR2 comprises the amino acid sequence set forth in SEQ ID NO: 26;
(3) the HCDR3 comprises the amino acid sequence set forth in SEQ ID NO: 27;
(4) the LCDR1 comprises the amino acid sequence set forth in SEQ ID NO: 28;
(5) the LCDR2 comprises the amino acid sequence set forth in SEQ ID NO: 29;
(6) the LCDR3 comprises the amino acid sequence set forth in SEQ ID NO: 30

In one embodiment, wherein,
(1) the HCDR1 comprises the amino acid sequence set forth in SEQ ID NO: 31;
(2) the HCDR2 comprises the amino acid sequence set forth in SEQ ID NO: 32;
(3) the HCDR3 comprises the amino acid sequence set forth in SEQ ID NO: 33;
(4) the LCDR1 comprises the amino acid sequence set forth in SEQ ID NO: 34;
(5) the LCDR2 comprises the amino acid sequence set forth in SEQ ID NO: 35;
(6) the LCDR3 comprises the amino acid sequence set forth in SEQ ID NO: 36

In one embodiment, wherein,
(1) the HCDR1 comprises the amino acid sequence set forth in SEQ ID NO: 37;
(2) the HCDR2 comprises the amino acid sequence set forth in SEQ ID NO: 38;
(3) the HCDR3 comprises the amino acid sequence set forth in SEQ ID NO: 39;
(4) the LCDR1 comprises the amino acid sequence set forth in SEQ ID NO: 40;
(5) the LCDR2 comprises the amino acid sequence set forth in SEQ ID NO: 41;
(6) the LCDR3 comprises the amino acid sequence set forth in SEQ ID NO: 42

In one embodiment, wherein, the heavy chain variable region comprises an amino acid sequence comprising at least 90%, at least 95%, or 100% sequence identity to the amino acid sequence set forth in SEQ ID NO: 43 or to SEQ ID NO: 43.

In one embodiment, wherein, the light chain variable region comprises the amino acid sequence set forth in SEQ ID NO: 44 or an amino acid sequence comprising at least 90%, at least 95%, or 100% sequence identity to SEQ ID NO: 44.

In one embodiment, wherein, the heavy chain variable region comprises the amino acid sequence set forth in SEQ ID NO: 45 or an amino acid sequence comprising at least 90%, at least 95% or more sequence identity to SEQ ID NO: 45.

In one embodiment, wherein, the light chain variable region comprises the amino acid sequence set forth in SEQ ID NO: 46 or an amino acid sequence comprising at least 90%, at least 95% or more sequence identity to SEQ ID NO: 46.

In one embodiment, wherein, the heavy chain variable region comprises the amino acid sequence set forth in SEQ ID NO: 47 or an amino acid sequence comprising at least 90%, at least 95% or more sequence identity to SEQ ID NO: 47.

In one embodiment, wherein, the light chain variable region comprises the amino acid sequence set forth in SEQ ID NO: 48 or an amino acid sequence comprising at least 90%, at least 95% or more sequence identity to SEQ ID NO: 48.

In one embodiment, wherein, the heavy chain variable region comprises the amino acid sequence set forth in SEQ ID NO: 49 or an amino acid sequence comprising at least 90%, at least 95% or more sequence identity to SEQ ID NO: 49.

In one embodiment, wherein, the light chain variable region comprises the amino acid sequence set forth in SEQ ID NO: 50 or an amino acid sequence comprising at least 90%, at least 95% or more sequence identity to SEQ ID NO: 50.

In one embodiment, wherein, the heavy chain variable region comprises the amino acid sequence set forth in SEQ ID NO: 51 or an amino acid sequence comprising at least 90%, at least 95% or more sequence identity to SEQ ID NO: 51.

In one embodiment, wherein, the light chain variable region comprises the amino acid sequence set forth in SEQ ID NO: 52 or an amino acid sequence comprising at least 90%, at least 95% or more sequence identity to SEQ ID NO: 52.

In one embodiment, wherein, the antibody is a humanized antibody.

The present invention provides an antibody or antigen-binding fragment, wherein, comprising a heavy chain variable region and/or a light chain variable region, wherein the heavy chain variable region comprises one of the amino acid sequences set forth in SEQ ID NO: 53 or SEQ ID NO: 54 or SEQ ID NO: 55 or SEQ ID NO: 56, and the light chain variable region comprises one of the amino acid sequences set forth in SEQ ID NO: 57 or SEQ ID NO: 58 or SEQ ID NO: 59.

The present invention provides an antibody or antigen-binding fragment, wherein, comprises a heavy chain variable region and/or a light chain variable region, wherein:
1) the heavy chain variable region comprises the amino acid sequence set forth in SEQ ID NO: 53, and the light chain variable region comprises the amino acid sequence set forth in SEQ ID NO: 57; or
2) the heavy chain variable region comprises the amino acid sequence set forth in SEQ ID NO: 53, and the light chain variable region comprises the amino acid sequence set forth in SEQ ID NO: 58; or
3) the heavy chain variable region comprises the amino acid sequence set forth in SEQ ID NO: 53, and the light chain variable region comprises the amino acid sequence set forth in SEQ ID NO: 59; or
4) the heavy chain variable region comprises the amino acid sequence set forth in SEQ ID NO: 54, and the light chain variable region comprises the amino acid sequence set forth in SEQ ID NO: 57; or
5) the heavy chain variable region comprises the amino acid sequence set forth in SEQ ID NO: 54, and the light chain variable region comprises the amino acid sequence set forth in SEQ ID NO: 58; or
6) the heavy chain variable region comprises the amino acid sequence set forth in SEQ ID NO: 54, and the light chain variable region comprises the amino acid sequence set forth in SEQ ID NO: 59; or
7) the heavy chain variable region comprises the amino acid sequence set forth in SEQ ID NO: 55, and the light chain variable region comprises the amino acid sequence set forth in SEQ ID NO: 57; or
8) the heavy chain variable region comprises the amino acid sequence set forth in SEQ ID NO: 55, and the light chain variable region comprises the amino acid sequence set forth in SEQ ID NO: 58; or
9) the heavy chain variable region comprises the amino acid sequence set forth in SEQ ID NO: 55, and the light chain variable region comprises the amino acid sequence set forth in SEQ ID NO: 59; or
10) the heavy chain variable region comprises the amino acid sequence set forth in SEQ ID NO: 56, and the light chain variable region comprises the amino acid sequence set forth in SEQ ID NO: 57; or
11) the heavy chain variable region comprises the amino acid sequence set forth in SEQ ID NO: 56, and the light chain variable region comprises the amino acid sequence set forth in SEQ ID NO: 58; or
12) the heavy chain variable region comprises the amino acid sequence set forth in SEQ ID NO: 56, and the light chain variable region comprises the amino acid sequence set forth in SEQ ID NO: 59.

In one embodiment, wherein, the heavy chain variable region comprises one of the amino acid sequences set forth in SEQ ID NO: 60 or SEQ ID NO: 61 or SEQ ID NO: 62 or SEQ ID NO: 63, and the light chain variable region comprises one of the amino acid sequences set forth in SEQ ID NO: 64 or SEQ ID NO: 65 or SEQ ID NO: 66.

In one embodiment, wherein, comprising a heavy chain variable region and/or a light chain variable region, wherein,
1) the heavy chain variable region comprises the amino acid sequence set forth in SEQ ID NO: 60, and the light chain variable region comprises the amino acid sequence set forth in SEQ ID NO: 64; or
2) the heavy chain variable region comprises the amino acid sequence set forth in SEQ ID NO: 60, and the light chain variable region comprises the amino acid sequence set forth in SEQ ID NO: 65; or
3) the heavy chain variable region comprises the amino acid sequence set forth in SEQ ID NO: 60, and the light chain variable region comprises the amino acid sequence set forth in SEQ ID NO: 66; or
4) the heavy chain variable region comprises the amino acid sequence set forth in SEQ ID NO: 61, and the light chain variable region comprises the amino acid sequence set forth in SEQ ID NO: 64; or
5) the heavy chain variable region comprises the amino acid sequence set forth in SEQ ID NO: 61, and the light chain variable region comprises the amino acid sequence set forth in SEQ ID NO: 65; or
6) the heavy chain variable region comprises the amino acid sequence set forth in SEQ ID NO: 61, and the light chain variable region comprises the amino acid sequence set forth in SEQ ID NO: 66; or
7) the heavy chain variable region comprises the amino acid sequence set forth in SEQ ID NO: 62, and the light chain variable region comprises the amino acid sequence set forth in SEQ ID NO: 64; or
8) the heavy chain variable region comprises the amino acid sequence set forth in SEQ ID NO: 62, and the light chain variable region comprises the amino acid sequence set forth in SEQ ID NO: 65; or
9) the heavy chain variable region comprises the amino acid sequence set forth in SEQ ID NO: 62, and the light chain variable region comprises the amino acid sequence set forth in SEQ ID NO: 66; or
10) the heavy chain variable region comprises the amino acid sequence set forth in SEQ ID NO: 63, and the light chain variable region comprises the amino acid sequence set forth in SEQ ID NO: 64; or
11) the heavy chain variable region comprises the amino acid sequence set forth in SEQ ID NO: 63, and the light chain variable region comprises the amino acid sequence set forth in SEQ ID NO: 65; or
12) the heavy chain variable region comprises the amino acid sequence set forth in SEQ ID NO: 63, and the light chain variable region comprises the amino acid sequence set forth in SEQ ID NO: 66.

In one embodiment, wherein, the heavy chain variable region comprises one of the amino acid sequences set forth in SEQ ID NO: 67 or SEQ ID NO: 68 or SEQ ID NO: 69 or SEQ ID NO: 70 or SEQ ID NO: 71 or SEQ ID NO: 72 or SEQ ID NO: 73 or SEQ ID NO: 74 and the light chain variable region comprises one of the amino acid sequences set forth in SEQ ID NO: 75 or SEQ ID NO: 76 or SEQ ID NO: 77 or SEQ ID NO: 78 or SEQ ID NO: 79 or SEQ ID NO: 80 or SEQ ID NO: 81.

In one embodiment, wherein, comprising a heavy chain variable region and/or a light chain variable region, wherein,
1) the heavy chain variable region comprises the amino acid sequence set forth in SEQ ID NO: 67, and the light chain variable region comprises the amino acid sequence set forth in SEQ ID NO: 75; Or
2) the heavy chain variable region comprises the amino acid sequence set forth in SEQ ID NO: 67, and the light chain variable region comprises the amino acid sequence set forth in SEQ ID NO: 76; Or
3) the heavy chain variable region comprises the amino acid sequence set forth in SEQ ID NO: 67, and the light chain variable region comprises the amino acid sequence set forth in SEQ ID NO: 77; Or
4) the heavy chain variable region comprises the amino acid sequence set forth in SEQ ID NO: 67, and the light chain variable region comprises the amino acid sequence set forth in SEQ ID NO: 78; Or
5) the heavy chain variable region comprises the amino acid sequence set forth in SEQ ID NO: 67 and the light chain variable region comprises the amino acid sequence set forth in SEQ ID NO: 79; Or
6) the heavy chain variable region comprises the amino acid sequence set forth in SEQ ID NO: 67, and the light chain variable region comprises the amino acid sequence set forth in SEQ ID NO: 80; Or
7) the heavy chain variable region comprises the amino acid sequence set forth in SEQ ID NO: 67, and the light chain variable region comprises the amino acid sequence set forth in SEQ ID NO: 81; Or
8) the heavy chain variable region comprises the amino acid sequence set forth in SEQ ID NO: 68 and the light chain variable region comprises the amino acid sequence set forth in SEQ ID NO: 75; Or
9) the heavy chain variable region comprises the amino acid sequence set forth in SEQ ID NO: 68, and the light chain variable region comprises the amino acid sequence set forth in SEQ ID NO: 76; Or
10) the heavy chain variable region comprises the amino acid sequence set forth in SEQ ID NO: 68, and the light chain variable region comprises the amino acid sequence set forth in SEQ ID NO: 77; Or
11) the heavy chain variable region comprises the amino acid sequence set forth in SEQ ID NO: 68, and the light chain variable region comprises the amino acid sequence set forth in SEQ ID NO: 78; Or
12) the heavy chain variable region comprises the amino acid sequence set forth in SEQ ID NO: 68, and the light chain variable region comprises the amino acid sequence set forth in SEQ ID NO: 79; Or
13) the heavy chain variable region comprises the amino acid sequence set forth in SEQ ID NO: 68, and the light chain variable region comprises the amino acid sequence set forth in SEQ ID NO: 80; Or
14) the heavy chain variable region comprises the amino acid sequence set forth in SEQ ID NO: 68, and the light chain variable region comprises the amino acid sequence set forth in SEQ ID NO: 81; Or
15) the heavy chain variable region comprises the amino acid sequence set forth in SEQ ID NO: 69, and the light chain variable region comprises the amino acid sequence set forth in SEQ ID NO: 75; Or
16) the heavy chain variable region comprises the amino acid sequence set forth in SEQ ID NO: 69, and the light chain variable region comprises the amino acid sequence set forth in SEQ ID NO: 76; Or
17) the heavy chain variable region comprises the amino acid sequence set forth in SEQ ID NO: 69, and the light chain variable region comprises the amino acid sequence set forth in SEQ ID NO: 77; Or
18) the heavy chain variable region comprises the amino acid sequence set forth in SEQ ID NO: 69, and the light chain variable region comprises the amino acid sequence set forth in SEQ ID NO: 78; Or
19) the heavy chain variable region comprises the amino acid sequence set forth in SEQ ID NO: 69, and the light chain variable region comprises the amino acid sequence set forth in SEQ ID NO: 79; Or
20) the heavy chain variable region comprises the amino acid sequence set forth in SEQ ID NO: 69, and the light chain variable region comprises the amino acid sequence set forth in SEQ ID NO: 80; Or
21) the heavy chain variable region comprises the amino acid sequence set forth in SEQ ID NO: 69, and the light chain variable region comprises the amino acid sequence set forth in SEQ ID NO: 81; Or
22) the heavy chain variable region comprises the amino acid sequence set forth in SEQ ID NO: 70, and the light chain variable region comprises the amino acid sequence set forth in SEQ ID NO: 75; Or
23) the heavy chain variable region comprises the amino acid sequence set forth in SEQ ID NO: 70, and the light chain variable region comprises the amino acid sequence set forth in SEQ ID NO: 76; Or
24) the heavy chain variable region comprises the amino acid sequence set forth in SEQ ID NO: 70, and the light chain variable region comprises the amino acid sequence set forth in SEQ ID NO: 77; Or
25) the heavy chain variable region comprises the amino acid sequence set forth in SEQ ID NO: 70, and the light chain variable region comprises the amino acid sequence set forth in SEQ ID NO: 78; Or
26) the heavy chain variable region comprises the amino acid sequence set forth in SEQ ID NO: 70, and the light chain variable region comprises the amino acid sequence set forth in SEQ ID NO: 79; Or
27) the heavy chain variable region comprises the amino acid sequence set forth in SEQ ID NO: 70, and the light chain variable region comprises the amino acid sequence set forth in SEQ ID NO: 80; Or
28) the heavy chain variable region comprises the amino acid sequence set forth in SEQ ID NO: 70, and the light chain variable region comprises the amino acid sequence set forth in SEQ ID NO: 81; Or
29) the heavy chain variable region comprises the amino acid sequence set forth in SEQ ID NO: 71, and the light chain variable region comprises the amino acid sequence set forth in SEQ ID NO: 75; Or
30) the heavy chain variable region comprises the amino acid sequence set forth in SEQ ID NO: 71, and the light chain variable region comprises the amino acid sequence set forth in SEQ ID NO: 76; Or
31) the heavy chain variable region comprises the amino acid sequence set forth in SEQ ID NO: 71, and the light chain variable region comprises the amino acid sequence set forth in SEQ ID NO: 77; Or
32) the heavy chain variable region comprises the amino acid sequence set forth in SEQ ID NO: 71, and the light chain variable region comprises the amino acid sequence set forth in SEQ ID NO: 78; Or
33) the heavy chain variable region comprises the amino acid sequence set forth in SEQ ID NO: 71, and the light chain variable region comprises the amino acid sequence set forth in SEQ ID NO: 79; Or
34) the heavy chain variable region comprises the amino acid sequence set forth in SEQ ID NO: 71, and the light chain variable region comprises the amino acid sequence set forth in SEQ ID NO: 80; Or
35) the heavy chain variable region comprises the amino acid sequence set forth in SEQ ID NO: 71, and the light chain variable region comprises the amino acid sequence set forth in SEQ ID NO: 81; Or
36) the heavy chain variable region comprises the amino acid sequence set forth in SEQ ID NO: 72, and the light chain variable region comprises the amino acid sequence set forth in SEQ ID NO: 75; Or
37) the heavy chain variable region comprises the amino acid sequence set forth in SEQ ID NO: 72, and the light chain variable region comprises the amino acid sequence set forth in SEQ ID NO: 76; Or
38) the heavy chain variable region comprises the amino acid sequence set forth in SEQ ID NO: 72 and the light chain variable region comprises the amino acid sequence set forth in SEQ ID NO: 77; Or
39) the heavy chain variable region comprises the amino acid sequence set forth in SEQ ID NO: 72, and the light chain variable region comprises the amino acid sequence set forth in SEQ ID NO: 78; Or
40) the heavy chain variable region comprises the amino acid sequence set forth in SEQ ID NO: 72, and the light chain variable region comprises the amino acid sequence set forth in SEQ ID NO: 79; Or
41) the heavy chain variable region comprises the amino acid sequence set forth in SEQ ID NO: 72, and the light chain variable region comprises the amino acid sequence set forth in SEQ ID NO: 80; Or
42) the heavy chain variable region comprises the amino acid sequence set forth in SEQ ID NO: 72, and the light chain variable region comprises the amino acid sequence set forth in SEQ ID NO: 81; Or
43) the heavy chain variable region comprises the amino acid sequence set forth in SEQ ID NO: 73, and the light chain variable region comprises the amino acid sequence set forth in SEQ ID NO: 75; Or
44) the heavy chain variable region comprises the amino acid sequence set forth in SEQ ID NO: 73, and the light chain variable region comprises the amino acid sequence set forth in SEQ ID NO: 76; Or
45) the heavy chain variable region comprises the amino acid sequence set forth in SEQ ID NO: 73, and the light chain variable region comprises the amino acid sequence set forth in SEQ ID NO: 77; Or
46) the heavy chain variable region comprises the amino acid sequence set forth in SEQ ID NO: 73, and the light chain variable region comprises the amino acid sequence set forth in SEQ ID NO: 78; Or
47) the heavy chain variable region comprises the amino acid sequence set forth in SEQ ID NO: 73, and the light chain variable region comprises the amino acid sequence set forth in SEQ ID NO: 79; Or
48) the heavy chain variable region comprises the amino acid sequence set forth in SEQ ID NO: 73, and the light chain variable region comprises the amino acid sequence set forth in SEQ ID NO: 80; Or
49) the heavy chain variable region comprises the amino acid sequence set forth in SEQ ID NO: 73, and the light chain variable region comprises the amino acid sequence set forth in SEQ ID NO: 81; Or
50) the heavy chain variable region comprises the amino acid sequence set forth in SEQ ID NO: 74, and the light chain variable region comprises the amino acid sequence set forth in SEQ ID NO: 75; Or
51) the heavy chain variable region comprises the amino acid sequence set forth in SEQ ID NO: 74, and the light chain variable region comprises the amino acid sequence set forth in SEQ ID NO: 76; Or
52) the heavy chain variable region comprises the amino acid sequence set forth in SEQ ID NO: 74, and the light chain variable region comprises the amino acid sequence set forth in SEQ ID NO: 77; Or
53) the heavy chain variable region comprises the amino acid sequence set forth in SEQ ID NO: 74, and the light chain variable region comprises the amino acid sequence set forth in SEQ ID NO: 78; Or
54) the heavy chain variable region comprises the amino acid sequence set forth in SEQ ID NO: 74, and the light chain variable region comprises the amino acid sequence set forth in SEQ ID NO: 79; Or
55) the heavy chain variable region comprises the amino acid sequence set forth in SEQ ID NO: 74, and the light chain variable region comprises the amino acid sequence set forth in SEQ ID NO: 80; Or
56) the heavy chain variable region comprises the amino acid sequence set forth in SEQ ID NO: 74, and the light chain variable region comprises the amino acid sequence set forth in SEQ ID NO: 81.

In one embodiment, wherein, the heavy chain variable region comprises one of the amino acid sequences set forth in SEQ ID NO: 82 or SEQ ID NO: 83 or SEQ ID NO: 84 or SEQ ID NO: 85 or SEQ ID NO: 86 or SEQ ID NO: 87 or SEQ ID NO: 88 or SEQ ID NO: 89 or SEQ ID NO: 90, and the light chain variable region comprises one of the amino acid sequences set forth in SEQ ID NO: 91 or SEQ ID NO: 92 or SEQ ID NO: 93 or SEQ ID NO: 94 or SEQ ID NO: 95 or SEQ ID NO: 96.

In one embodiment, wherein, comprises a heavy chain variable region and/or a light chain variable region, wherein,
1) the heavy chain variable region comprises the amino acid sequence set forth in SEQ ID NO: 82, and the light chain variable region comprises the amino acid sequence set forth in SEQ ID NO: 91; Or
2) the heavy chain variable region comprises the amino acid sequence set forth in SEQ ID NO: 82, and the light chain variable region comprises the amino acid sequence set forth in SEQ ID NO: 92; Or
3) the heavy chain variable region comprises the amino acid sequence set forth in SEQ ID NO: 82, and the light chain variable region comprises the amino acid sequence set forth in SEQ ID NO: 93; Or
4) the heavy chain variable region comprises the amino acid sequence set forth in SEQ ID NO: 82, and the light chain variable region comprises the amino acid sequence set forth in SEQ ID NO: 94; Or
5) the heavy chain variable region comprises the amino acid sequence set forth in SEQ ID NO: 82, and the light chain variable region comprises the amino acid sequence set forth in SEQ ID NO: 95; Or
6) the heavy chain variable region comprises the amino acid sequence set forth in SEQ ID NO: 82, and the light chain variable region comprises the amino acid sequence set forth in SEQ ID NO: 96; Or
7) the heavy chain variable region comprises the amino acid sequence set forth in SEQ ID NO: 83, and the light chain variable region comprises the amino acid sequence set forth in SEQ ID NO: 91; Or
8) the heavy chain variable region comprises the amino acid sequence set forth in SEQ ID NO: 83, and the light chain variable region comprises the amino acid sequence set forth in SEQ ID NO: 92; Or
9) the heavy chain variable region comprises the amino acid sequence set forth in SEQ ID NO: 83, and the light chain variable region comprises the amino acid sequence set forth in SEQ ID NO: 93; Or
10) the heavy chain variable region comprises the amino acid sequence set forth in SEQ ID NO: 83, and the light chain variable region comprises the amino acid sequence set forth in SEQ ID NO: 94; Or
11) the heavy chain variable region comprises the amino acid sequence set forth in SEQ ID NO: 83, and the light chain variable region comprises the amino acid sequence set forth in SEQ ID NO: 95; Or
12) the heavy chain variable region comprises the amino acid sequence set forth in SEQ ID NO: 83, and the light chain variable region comprises the amino acid sequence set forth in SEQ ID NO: 96; Or
13) the heavy chain variable region comprises the amino acid sequence set forth in SEQ ID NO: 84, and the light chain variable region comprises the amino acid sequence set forth in SEQ ID NO: 91; Or
14) the heavy chain variable region comprises the amino acid sequence set forth in SEQ ID NO: 84, and the light chain variable region comprises the amino acid sequence set forth in SEQ ID NO: 92; Or
15) the heavy chain variable region comprises the amino acid sequence set forth in SEQ ID NO: 84, and the light chain variable region comprises the amino acid sequence set forth in SEQ ID NO: 93; Or
16) the heavy chain variable region comprises the amino acid sequence set forth in SEQ ID NO: 84, and the light chain variable region comprises the amino acid sequence set forth in SEQ ID NO: 94; Or
17) the heavy chain variable region comprises the amino acid sequence set forth in SEQ ID NO: 84, and the light chain variable region comprises the amino acid sequence set forth in SEQ ID NO: 95; Or
18) the heavy chain variable region comprises the amino acid sequence set forth in SEQ ID NO: 84, and the light chain variable region comprises the amino acid sequence set forth in SEQ ID NO: 96; Or
19) the heavy chain variable region comprises the amino acid sequence set forth in SEQ ID NO: 85, and the light chain variable region comprises the amino acid sequence set forth in SEQ ID NO: 91; Or
20) the heavy chain variable region comprises the amino acid sequence set forth in SEQ ID NO: 85, and the light chain variable region comprises the amino acid sequence set forth in SEQ ID NO: 92; Or
21) the heavy chain variable region comprises the amino acid sequence set forth in SEQ ID NO: 85, and the light chain variable region comprises the amino acid sequence set forth in SEQ ID NO: 93; Or
22) the heavy chain variable region comprises the amino acid sequence set forth in SEQ ID NO: 85, and the light chain variable region comprises the amino acid sequence set forth in SEQ ID NO: 94; Or
23) the heavy chain variable region comprises the amino acid sequence set forth in SEQ ID NO: 85, and the light chain variable region comprises the amino acid sequence set forth in SEQ ID NO: 95; Or
24) the heavy chain variable region comprises the amino acid sequence set forth in SEQ ID NO: 85, and the light chain variable region comprises the amino acid sequence set forth in SEQ ID NO: 96; Or
25) the heavy chain variable region comprises the amino acid sequence set forth in SEQ ID NO: 86, and the light chain variable region comprises the amino acid sequence set forth in SEQ ID NO: 91; Or
26) the heavy chain variable region comprises the amino acid sequence set forth in SEQ ID NO: 86, and the light chain variable region comprises the amino acid sequence set forth in SEQ ID NO: 92; Or
27) the heavy chain variable region comprises the amino acid sequence set forth in SEQ ID NO: 86, and the light chain variable region comprises the amino acid sequence set forth in SEQ ID NO: 93; Or
28) the heavy chain variable region comprises the amino acid sequence set forth in SEQ ID NO: 86, and the light chain variable region comprises the amino acid sequence set forth in SEQ ID NO: 94; Or
29) the heavy chain variable region comprises the amino acid sequence set forth in SEQ ID NO: 86, and the light chain variable region comprises the amino acid sequence set forth in SEQ ID NO: 95; Or
30) the heavy chain variable region comprises the amino acid sequence set forth in SEQ ID NO: 86, and the light chain variable region comprises the amino acid sequence set forth in SEQ ID NO: 96; Or
31) the heavy chain variable region comprises the amino acid sequence set forth in SEQ ID NO: 87, and the light chain variable region comprises the amino acid sequence set forth in SEQ ID NO: 91; Or
32) the heavy chain variable region comprises the amino acid sequence set forth in SEQ ID NO: 87, and the light chain variable region comprises the amino acid sequence set forth in SEQ ID NO: 92; Or
33) the heavy chain variable region comprises the amino acid sequence set forth in SEQ ID NO: 87, and the light chain variable region comprises the amino acid sequence set forth in SEQ ID NO: 93; Or
34) the heavy chain variable region comprises the amino acid sequence set forth in SEQ ID NO: 87, and the light chain variable region comprises the amino acid sequence set forth in SEQ ID NO: 94; Or
35) the heavy chain variable region comprises the amino acid sequence set forth in SEQ ID NO: 87, and the light chain variable region comprises the amino acid sequence set forth in SEQ ID NO: 95; Or
36) the heavy chain variable region comprises the amino acid sequence set forth in SEQ ID NO: 87, and the light chain variable region comprises the amino acid sequence set forth in SEQ ID NO: 96; Or
37) the heavy chain variable region comprises the amino acid sequence set forth in SEQ ID NO: 88, and the light chain variable region comprises the amino acid sequence set forth in SEQ ID NO: 91; Or
38) the heavy chain variable region comprises the amino acid sequence set forth in SEQ ID NO: 88, and the light chain variable region comprises the amino acid sequence set forth in SEQ ID NO: 92; Or
39) the heavy chain variable region comprises the amino acid sequence set forth in SEQ ID NO: 88, and the light chain variable region comprises the amino acid sequence set forth in SEQ ID NO: 93; Or
40) the heavy chain variable region comprises the amino acid sequence set forth in SEQ ID NO: 88, and the light chain variable region comprises the amino acid sequence set forth in SEQ ID NO: 94; Or
41) the heavy chain variable region comprises the amino acid sequence set forth in SEQ ID NO: 88, and the light chain variable region comprises the amino acid sequence set forth in SEQ ID NO: 95; Or
42) the heavy chain variable region comprises the amino acid sequence set forth in SEQ ID NO: 88, and the light chain variable region comprises the amino acid sequence set forth in SEQ ID NO: 96; Or
43) the heavy chain variable region comprises the amino acid sequence set forth in SEQ ID NO: 89, and the light chain variable region comprises the amino acid sequence set forth in SEQ ID NO: 91; Or
44) the heavy chain variable region comprises the amino acid sequence set forth in SEQ ID NO: 89, and the light chain variable region comprises the amino acid sequence set forth in SEQ ID NO: 92; Or
45) the heavy chain variable region comprises the amino acid sequence set forth in SEQ ID NO: 89, and the light chain variable region comprises the amino acid sequence set forth in SEQ ID NO: 93; Or
46) the heavy chain variable region comprises the amino acid sequence set forth in SEQ ID NO: 89, and the light chain variable region comprises the amino acid sequence set forth in SEQ ID NO: 94; Or
47) the heavy chain variable region comprises the amino acid sequence set forth in SEQ ID NO: 89, and the light chain variable region comprises the amino acid sequence set forth in SEQ ID NO: 95; Or
48) the heavy chain variable region comprises the amino acid sequence set forth in SEQ ID NO: 89, and the light chain variable region comprises the amino acid sequence set forth in SEQ ID NO: 96; Or
49) the heavy chain variable region comprises the amino acid sequence set forth in SEQ ID NO: 90, and the light chain variable region comprises the amino acid sequence set forth in SEQ ID NO: 91; Or
50) the heavy chain variable region comprises the amino acid sequence set forth in SEQ ID NO: 90, and the light chain variable region comprises the amino acid sequence set forth in SEQ ID NO: 92; Or
51) the heavy chain variable region comprises the amino acid sequence set forth in SEQ ID NO: 90, and the light chain variable region comprises the amino acid sequence set forth in SEQ ID NO: 93; Or
52) the heavy chain variable region comprises the amino acid sequence set forth in SEQ ID NO: 90, and the light chain variable region comprises the amino acid sequence set forth in SEQ ID NO: 94; Or
53) the heavy chain variable region comprises the amino acid sequence set forth in SEQ ID NO: 90, and the light chain variable region comprises the amino acid sequence set forth in SEQ ID NO: 95; Or
54) the heavy chain variable region comprises the amino acid sequence set forth in SEQ ID NO: 90, and the light chain variable region comprises the amino acid sequence set forth in SEQ ID NO: 96.

In addition, the present invention provides a fully humanized antibody or antigen-binding fragment, whererin, further comprising an Fc region, optionally that of a human immunoglobulin (Ig), or optionally that of a human IgG, whereas the Fc region is derived from human IgG1, IgG2, IgG3, IgG4, IgA1, IgA2 or IgM

The antigen-binding fragment in the present invention is scFv, scFv-Fc, Fv, Fab, Fab ', F (ab)₂ or F (ab ')₂.

Optionally, the antigen-binding fragment in the present invention is F (ab)₂, Fab or scFv.

F(ab)₂ fragments are "bivalent". "Bivalent" means that the antibodies and F(ab')2 fragments have two antigen combining sites. In contrast, Fab, Fv, and scFv fragments are monovalent, comprising only one antigen-binding site.

F(ab)₂ is an antibody fragment comprising a molecular weight of about 50,000 and comprising antigen-binding activity among fragments obtained by treating an IgG antibody molecule with the protease papain (cleaving the amino acid residue at position 224 of the H chain), wherein about half of the N-terminal fragment and the entire L chain are held together by disulfide bonds (C.A.K Borrebaeck, editor (1995) Antibody Engineering (Breakthroughs in Molecular Biology), Oxford University Press; R.Kontermann & S.Duebel, editors (2001) Antibody Engineering (Springer Laboratory Manual), Springer Verlag).

Fab is an antibody fragment comprising a molecular weight of about 50,000 and comprising antigen-binding activity obtained by cleaving the disulfide bond in the hinge region of the above-mentioned F(ab')₂. The Fab of the invention can be produced by treating F(ab)₂ with a reducing agent such as dithiothreitol.

"Single-chain Fv" or "scFv" means a molecule comprising an antibody heavy chain variable domain (or region; VH) and an antibody light chain variable domain (or region; VL) connected by a linker. Such scFv molecules may comprise the general structure: H2-VL-linker-VH-COOH or H2-VH-linker-VL-COOH. Suitable prior art linkers consist of the repeated GGGGS amino acid sequence or variants thereof, for example using 1-4 repeat variants.

The antibody or antigen-binding fragment in the present invention is a monoclonal antibody, a bispecific antibody, a multispecific antibody, a recombinant antibody, a chimeric antibody, a divalent antibody, an anti-idiotypic antibody, or a fusion protein.

Besides, the present invention also provides an antibody-drug conjugate comprising the aforementioned antibody or antigen-binding fragment, and an additional effector molecule conjugate.

Besides, the present invention also provides a nucleic acid molecule encoding the aforementioned antibody or antigen-binding fragment.

The nucleic acid molecule of the present invention, a promoter is operably linked.

In addition, the present invention also provides a recombinant expression vector, which comprises the aforementioned nucleic acid molecule of the present invention.

In addition, the present invention also provides a plasmid containing the nucleic acid molecule of the present invention.

In addition, the present invention also provides a host cell containing the nucleic acid molecule of the present invention, the recombinant expression vector of the present invention or the plasmid of the present invention.

In addition, the present invention also provides a method for detecting L1CAM expression in a biological sample, comprising the following steps: contacting the biological sample with any of the aforementioned antibodies or antigen-binding fragments thereof of the present invention, and detecting the expression of the immune The presence or absence of the complex, wherein the presence of the immune complex indicates the presence of L1CAM in the biological sample.

In addition, the present invention also provides a pharmaceutical composition, including any antibody or antigen-binding fragment thereof of the present invention. The antibody-drug conjugate of the present invention act as an active substance, and a pharmaceutically acceptable carrier.

The present invention provides a pharmaceutical composition, an antibody or antigen-binding fragment or bispecific antibody or chimeric antigen receptor of the present invention or a nucleic acid molecule thereof, a plasmid or a cell expressing the chimeric antigen receptor, and physiologically or pharmaceutically acceptable carrier, excipient or stabilizer. The composition includes but not limited to freeze-dried dosage form, aqueous solution dosage form, liposome or capsule, etc. The concentration of the monoclonal antibody or antigen-binding fragment or bispecific antibody or chimeric antigen receptor or nucleic acid molecule thereof, plasmid or cell expressing the chimeric antigen receptor of the invention can vary from about 0.1% to 100% (weight).

In addition, the present invention also provides a kit, including any one of the aforementioned antibodies or antigen-binding fragments of the present invention, the antibody-drug conjugates described in the present invention and/or the antibody-drug conjugates described in the present invention pharmaceutical composition.

In addition, the present invention also provides a method for treating cancer, which includes administering any one of the aforementioned antibodies or antigen-binding fragments thereof, the antibody-drug conjugates described in the present invention to a subject in need thereof. or the pharmaceutical composition of the present invention.

In the aforementioned method of the present invention, it also includes detecting the presence of L1-CAM in the cancer sample before administering the drug.

Preferably, the antibodies or antigen-binding fragments of the present invention are fully humanized. The said fully humanized is an antibody or an antigen-binding fragment thereof is defined herein is non-chimeric (e.g., non-humanized) and does not come from non-humanized species (whether the whole is still part). fully human antibody or antigen-binding fragment thereof can be derived from human, or can be synthetic human antibody. Another example of a fully human antibody or an antigen-binding fragment thereof is a human antibody encoded by nucleic acid isolated from a library of antibody sequences of human origin (e.g., based on such a library of antibodies taken from natural sources of humans).

The antibody or antigen-binding fragment thereof of the invention, which is a monoclonal antibody, bispecific antibody, multi-specific antibody, recombinant antibody, chimeric antibody, divalent antibody, anti-idiotypic antibody or fusion protein.

The invention also provides an antibody or antigen binding fragment capable of binding L1CAM, which is a bispecific antibody or a bispecific antigen-binding fragment. In some embodiments, the bispecific antibody or bispecific antigen-binding fragment may be isolated. The term "bispecific" means that the antigen-binding molecule is capable of specifically binding to at least two different antigenic determinants. The bispecific antibodies and bispecific antigen-binding fragments comprise an antigen-binding fragment according to the present invention. In some embodiments, the bispecific antibodies and bispecific antigen binding fragments comprise an antigen binding fragment capable of binding to L1CAM, wherein the antigen binding fragment capable of binding to L1CAM comprises an antigen binding fragment according to the present invention. In some embodiments, the bispecific antibodies and bispecific antigen binding fragments comprise an antigen binding fragment capable of binding to L1CAM, and an antigen binding fragment capable of binding to another target protein. In some embodiments, the bispecific antibody or antigen-binding fragment comprises an antigen-binding molecule capable of binding L1CAM and an antigen-binding molecule capable of binding other than L1CAM. The antigen other than L1CAM is an immune cell surface molecule. In some embodiments, the antigen other than L1CAM is a cancer cell antigen In some embodiments, the antigen other than L1CAM is a receptor molecule, e.g., a cell surface receptor.

The present invention provides a nucleic acid encoding said monoclonal antibody or antigen-binding fragment or bispecific antibody or chimeric antigen receptor. The present invention provides a plasmid comprising the nucleic acid, optionally operably linked to regulatory sequences such as promoters, enhancers and the like. The invention provides host cells comprising the plasmid, and methods for producing and optionally recovering the monoclonal antibody or antigen-binding fragment or bispecific antibody or chimeric antigen receptor. The host cell of the present invention can be any prokaryotic cell or eukaryotic cell, including but not limited to bacterial cells (such as Escherichia coli, Bacillus subtilis), insect cells (such as using a baculovirus expression system), yeast or mammalian cells (such as CHO or BHK cell lines). Other suitable host cells are known to those skilled in the art.

Preferably, the disease described in the present invention is cancer, including but not limited to lymphoma, blastoma, sarcoma (including liposarcoma), neuroendocrine tumor, mesothelioma, schwannoma, meningioma, adenoma, melanoma and nonleukemic leukemia or lymphoid malignancies. More specific examples of the aforementioned cancers include squamous cell carcinoma (e.g., squamous cell carcinoma), lung cancer, small cell lung cancer, non-small cell lung cancer, lung adenocarcinoma, and lung squamous cell carcinoma, peritoneal carcinoma, hepatocellular carcinoma, gastric cancer , gastrointestinal cancer, pancreatic cancer, malignant glioma, cervical cancer, ovarian cancer, liver cancer, bladder cancer, hepatoma, breast cancer, colon cancer, rectal cancer, colorectal cancer, endometrial or uterine cancer, salivary gland cancer , kidney cancer, prostate cancer, vulvar cancer, thyroid cancer, liver cancer, anal cancer, penis cancer, testicular cancer, esophagus cancer, bile duct tumor, head cancer, neck cancer, bone marrow stromal tumor, osteoclastoma, multiple myeloma, Osteolytic bone cancers, central nervous system tumors, brain tumors (glioma, neuroblastoma, astrocytoma, medulloblastoma, ependymoma, and retinoblastoma) , nasopharyngeal carcinoma, basal cell carcinoma, cholangiocarcinoma, Kaposi's sarcoma, primary liver or endometrial carcinoma, and tumors of the vasculature (angiosarcoma and hemagiopericytoma), hematologic neoplasms, Hodgkin's lymphoma, non-Hodgkin's lymphoma (Burkitt's lymphoma, small lymphocytic lymphoma/chronic lymphocytic leukemia, mycosis fungoides, mantle cell lymphoma, follicular lymphoma, diffuse giant B-cell lymphoma, marginal zone lymphoma, hairy cell leukemia, and lymphoplasmacytic leukemia), lymphocyte precursor cell neoplasms, B-cell acute lymphoblastic nonleukemic leukemia/lymphoma, T-cell acute Lymphoblastic nonleukemic leukemia/lymphoma, thymoma, mature T and NK cell neoplasms, peripheral T cell nonleukemic leukemia, mature T cell nonleukemic leukemia/T cell lymphoma, large granular lymphocytic leukemia , Langerhans cell histiocytosis, myeloma in acute myeloid leukemia, mature acute myeloid leukemia (AML), differentiated acute myeloid leukemia, acute promyelocytic leukemia, acute myelomonocytic leukemia, acute monocytic leukemia, myelodysplastic syndrome, chronic myeloproliferative disease, chronic myelogenous leukemia, hematologic malignancies such as multiple myeloma (MM), myelodysplastic syndrome (MDS) and acute myeloid leukemia (AML).

The antibodies or antigen-binding fragments or bispecific antibodies or chimeric antigen receptors or nucleic acid molecules thereof, plasmids or cells expressing the chimeric antigen receptors of the present invention, and pharmaceutical compositions can be administered through various routes For human or animal subjects, the route of administration generally depends on the characteristics of the disease to be treated itself. In general, the methods of the present invention may be practiced using any mode of administration that is medically acceptable, including oral, rectal, topical, intraocular, intracisternal, intracerebroventricular, intratracheal, intranasal drops Injection, transdermal, subcutaneous, intrathecal, intramuscular, intraperitoneal, intraperitoneal, intracranial infusion or intravenous infusion.

In order that the invention may be more thoroughly understood, some definitions are listed below. The above definitions are intended to include grammatical equivalents.

As used herein, "antibody" refers to a protein consisting of one or more polypeptides encoded by substantially all or part of a recognized immunoglobulin gene. the recognized immunoglobulin gene, such as in a human, comprising kappa (κ), lambda (lambda) and a heavy chain locus, wherein comprising a plurality of variable region gene, and the isotype constant region gene mu (µ) delta (delta), gamma (gamma), epsilon (epsilon), alpha (alpha) respectively encoding IgM, IgD, IgG, IgE and IgA. Antibodies herein are meant to include full-length antibodies and antibody fragments, as well as natural antibodies from any organism, engineered antibodies, or recombinantly produced antibodies for experimental, therapeutic or other purposes as further specified below. The term "antibody" includes antibody fragments, known in the art, such as Fab, Fab', F(ab')2, Fv, scFv, or other antigen-binding subsequences of antibodies, or by modifying intact antibodies or using recombinant DNA techniques Antibody fragments produced by de novo synthesis of those antibodies. The term "antibody" includes monoclonal as well as polyclonal antibodies. Antibodies can be antagonists, agonists, neutralizing antibodies, or inhibitory antibodies, or stimulating antibodies. Antibodies of the invention may be non-human antibodies, chimeric antibodies, humanized antibodies or fully human antibodies.

The term "monoclonal antibody" as used herein refers to an antibody that is obtained from a substantially homogeneous antibody population, i.e., the population comprising a single antibody may be the same in addition to possible mutations (e.g., natural mutations) that may be present in a very small amount. Thus, the term "monoclonal" indicates the nature of the antibody, i.e., not a mixture of non-related (discrete) antibodies. In contrast to the polyclonal antibody preparations that typically include different antibodies directed against different determinants (epitopes), each monoclonal antibody of the monoclonal antibody formulation is directed against a single determinant on the antigen. In addition to their specificity, monoclonal antibody preparations are advantageous in that they are not normally contaminated by other immunoglobulins. The term "monoclonal" should not be understood to require the production of the antibodies by any particular method. The term monoclonal antibody specifically comprises a fully human antibody. (refCN201280067865-antibody of anti-FGFR2 and application-adjudication grant)

As used herein, the term "complementarity determining region" (CDR, e.g., CDR1, CDR2, and CDR3) refers to an amino acid residue of an antibody variable region, and the presence of complementarity determining regions is necessary for antigen binding. The "antigen-binding region" of the antibody is usually present in one or more hypervariable regions of the antibody, for example, CDR regions of CDR1, CDR2 and CDR3. each complementarity determining region may comprise amino acid residues from the complementarity determining region as defined by Kabat, for example, residues 24-34 (L1), 50-56 (L2) and 89-97 (L3) in the light chain variable region and 26-33 (H1) in the heavy chain variable region, 51-57 (H2) and 96-105 (H3) (Kabat etal, Sequences of Proteins of Immulological Interest, 5th Ed.Public Health Service, National Institutes of Health, 1991).

"Antigen" as used herein means a compound, composition or substance that can stimulate antibody production or T cell response in an animal, including compositions injected or absorbed into the animal, which can be a protein, carbohydrate, lipid or other Pathogens.

The "functional fragment" or "antigen-binding fragment" of an antibody/immunoglobulin is defined herein as a fragment of an antibody/immunoglobulin that retains the antigen-binding region (e.g., a variable region of IgG). The "antigen-binding region" of the antibody is usually present in one or more hypervariable regions of the antibody, e.g., CDR1, CDR-2 and/or CDR-3 regions; However, the variable "framework" region may also play an important role in antigen binding, for example, by providing a framework for a CDR. Preferably, the antigen-binding region comprises at least amino acid residue of variable light (VL) 4-103 and amino acid residue of variable heavy (VH) chain 5-109 5-109, more preferably VL amino acid residue 3-107 and VH of amino acid residue 4-111; and particularly preferably a complete VL and VH chain (VL amino acid first 1-109 and VH amino acid position 1-113); No. WO 97/08320). A preferred class of immunoglobulins for use in the present invention are IgG.

As used herein, "amino acid" means one or any non-natural analog of 20 naturally occurring amino acids, which may be located at a specific specified position. The term "protein" refers to at least two covalently linked amino acids, which include proteins, polypeptides, oligopeptides and peptides. The protein can be composed of naturally occurring amino acid and peptide bond, or composed of synthetic peptide mimetic structure, the peptide mimetic is analogue. As such, "amino acid" or "peptide residue" as used herein means a naturally occurring and synthetic amino acid. For example, for purposes of the present invention, homophenylalanine, citrulline and norleucine are considered to be amino acids for the purposes of the present invention. "Amino acid" also includes a subamino acid residue such as proline and hydroxyproline. The side chain may be (R) or (S) configuration. In a preferred embodiment, the amino acid is present in the (S) or L-configuration. If a non-naturally occurring side chain is used, a non-amino acid substitution may be used, for example, to prevent or delay degradation in vivo.

As used herein, "Identity" means similarity between nucleotide or amino acid sequences, otherwise known as sequence identity. Sequence identity is usually measured in terms of percent identity (or similarity or homology); the higher the percent, the more similar the two sequences are. Homologs or variants will have a relatively high degree of sequence identity when aligned using standard methods. Sequence alignment methods for comparison are well known in the art. Various procedures and alignment algorithms are described in: Smith and Waterman, Adv Appl. Math., 2: 482, 1981; Needlema and Wunsch, J. Mol. Biol. 48: 443, 1970; Pearson and Lipman, Proc. Natl. Acad. Sci. U.S.A. 85:2444, 1988; Higgins and Sharp, Gene 73: 237-244, 1988; Higgins and Sharp, CABIOS 5: 151-153, 1989; Corpetet. al, Nucleic Acids Research 16: 10881-10890,1988; and Altschul et. al, Nature Genet., 1994, 6: 119-129_{∘}

The NCBI Basic Local Alignment Search Tool (BLAST^{™}) (Altschul et al., J. Mol. Biol., 215:403-410, 1990.) is available from several sources, including the National Center for Biotechnology Information (NCBI, Bethesda, Md.) and are available on the Internet for the sequence analysis programs blastp, blastn, blastx, tblastn and tblastx.

As used herein, "nucleic acid" means a polymer formed by phosphodiester bond between nucleotide units (ribonucleotides, deoxyribonucleotides, related naturally occurring structural variants and synthetic non-naturally occurring analogs thereof). Thus, the term includes nucleotide polymers in which the nucleotides and the bonds between them include non-naturally occurring synthetic analogs such as, but not limited to, phosphorothioates, phosphoramidates, methyl phosphates, chiral Methyl phosphate, 2'-O-methylribonucleotide, peptide nucleic acid (PNA), etc. For example, these polynucleotides can be synthesized using an automated DNA synthesizer. The term "oligonucleotide" generally refers to short polynucleotides, usually no more than about 50 nucleotides. It will be understood that when a nucleotide sequence is represented by a DNA sequence (i.e. A, T, G, C), this also includes an RNA sequence in which "U" replaces "T" (i.e. A, U, G, C).

Conventional symbols are used herein to describe nucleotide sequences: the left-hand 5' end of a single-stranded nucleotide sequence; the left-hand direction of a double-stranded nucleotide sequence is called the 5' direction. The direction in which 5' to 3' nucleotides added to the nascent RNA transcript is called the direction of transcription. The DNA strand with the same sequence as the mRNA is called the coding strand.

As used herein, "encoding" refers to the intrinsic properties of specific nucleotide sequences in polynucleotides, such as genes, cDNA or mRNA, used as templates for the synthesis of other polymers and macromolecules in biological processes with defined nucleotide sequences, or defined amino acid sequences and resulting biological properties. Thus, if the transcription and translation of mRNA produced by that gene produces a protein in a cell or other biological system, the gene encodes for a protein. Coding strands (whose nucleotide sequence is identical to the mRNA sequence and are usually provided in the sequence list) and non-coding strands (used as transcription templates, genes, or cDNA) can be referred to as coding proteins, or other products of that gene or cDNA. Unless otherwise stated, "nucleotide sequences encoding amino acid sequences" includes all nucleotide sequences that are degenerate with each other and encode the same amino acid sequence. Nucleotide sequences encoding proteins and RNA may include introns.

As used herein, the "plasmid" used herein refers to a plasmid artificially constructed based on a natural plasmid to adapt to laboratory operations. Nucleic acid molecules can be introduced into a host cell, thereby producing a transformed host cell. The vector may include nucleic acid sequences that permit its replication in the host cell, such as an origin of replication, and may also include one or more selectable marker genes and other genetic elements known in the art.

The term "host cell" as used herein, also known as recipient cell, refers to a host cell that receives an exogenous gene in transformation and transduction (infection).

As used herein, "pharmaceutically acceptable carrier" means a conventional pharmaceutically acceptable vehicle. Remington's Pharmaceutical Sciences, EWMartin, Mack Publishing Co., Easton, Pa., 15th edition (1975), describing compositions and formulations suitable for drug delivery of one or more therapeutic compounds or molecules (e.g., one or more antibodies), and additional agents.

As used herein, the term "diagnostic" disease refers to the determination of the patient's condition and its development after examination of the patient. "Preventing" a disease means inhibiting the complete development of the disease. "Treatment" means a therapeutic intervention that improves the signs or symptoms of a disease or pathological condition after it begins to develop.

As used herein, "Administering" means selecting the appropriate route to introduce said substance into the subject. For example, if the chosen route is intravenous, the composition is administered by introducing said substance into the subject's vein.

As used herein, "effective preventive/therapeutic dose" means a specific agent sufficient to achieve the desired effect in subjects treated with this agent. The exact dose will depend on the purpose of treatment and may be determined by those skilled in the art through the use of well-known techniques. Doses range from 0.01 to 100 mg/kg body weight or larger, such as 0.1, 1, 10, or 50 mg/kg body weight, preferably 1-10 mg/kg. As well known in the art, for antibody or Fc fusion degradation, systemic or local drug delivery and rate of new protease synthesis, as well as age, weight, general health status, sex, diet, timing of administration, drug interactions, and severity of the condition, adjustments may be necessary and may be determined by those skilled in the art by conventional experimental methods. Such reagents include monomeric Fc domain molecules described herein. In a non-limiting instance, this can be the amount of HIV-specific monomer Fc domains (or HIV-specific CH3 domain molecules) used to prevent, treat, or improve HIV infection. Ideally, a therapeutically effective amount of antibody is an amount sufficient to prevent, treat, or improve infection or disease, such as caused by HIV infection in the subject, without causing a significant cytotoxic effect in the subject. The treatment-effective amount of the agent used for prevention, improvement and / or treatment of subjects will depend on the subject being treated, the type and severity of suffering, and the manner in which the therapeutic composition is administered.

As used herein, "cancer" is a solid tumor or a bloodborne cancer. The solid tumor described in the present invention is sarcoma or carcinoma, such as fibrosarcoma, myxosarcoma, liposarcoma, chondrosarcoma, osteosarcoma, or another sarcoma, synovoma, mesothelioma, Ewing tumor, leiomyosarcoma, rhabdomyosarcoma, colon cancer, lymphoid malignancy, pancreatic cancer, breast cancer, lung cancer, ovarian cancer, prostate cancer, hepatocellular carcinoma, squamous cell carcinoma, basal cell carcinoma, adenocarcinoma, sweat gland carcinoma, sebaceous adenocarcinoma, papillary carcinoma, papillary adenocarcinoma, medullary carcinoma, bronchial carcinoma, renal cell carcinoma, hepatocellular carcinoma, Cholangiocarcinoma, choriocarcinoma, Wilms tumor, cervical cancer, testicular tumor, bladder cancer, or central nervous system tumor (eg, glioma, astrocytoma, medulloblastoma, craniopharyngioma, ependymoma, pineal gland, angioblastoma, acoustic neuroma, oligodendroglioma, hemangioma, melanoma, neuroblastoma, or retinoblastoma). The bloodborne cancer described in the present invention is leukemia, such as acute leukemia (such as acute lymphoblastic leukemia, acute myeloid leukemia, acute myeloid leukemia and myeloblasts, promyelocyte, myeloid monocytes, monocytes and erythrocyte); Chronic leukemia (e.g. chronic myelogenous (granulocytic) leukemia, chronic myeloid leukemia and chronic lymphocytic leukemia), polycythemia vera, lymphoma, Hodgkin's disease, non-Hodgkin lymphoma (indolent and advanced form), multiple myeloma, Waldenstrom macroglobulinemia, heavy chain diseases, myelodysplastic syndrome, hirsutistic leukemia or myelodysplastic disorders.

Unless defined otherwise, all technical and scientific terms used herein have the same meaning as commonly understood the ordinary skilled person in the technical field. The singular terms "a", "an" and "the" include plural referents unless the context clearly dictates otherwise. It is also to be understood that all base sizes or amino acid sizes, and all molecular weight or molecular weight values given for nucleic acids or polypeptides are approximate and are provided for purposes of description. Although methods and materials similar or equivalent to those described herein can be used in the practice or testing of the present disclosure, suitable methods and materials are described below. The term "comprising" means "including". All publications, patent applications, patents, and other references mentioned herein are incorporated by reference in their entirety. In the event of conflict, this specification (including explanation of terms) will control. In addition, the materials, methods, and examples are illustrative only and not limiting.

### Description of the drawings

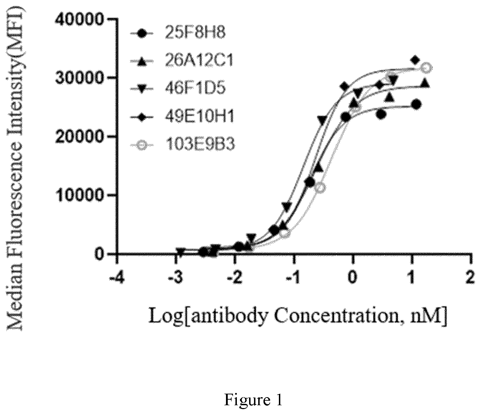

Figure 1, Binding curve of mouse anti-L1CAM monoclonal antibody to HeLa cells

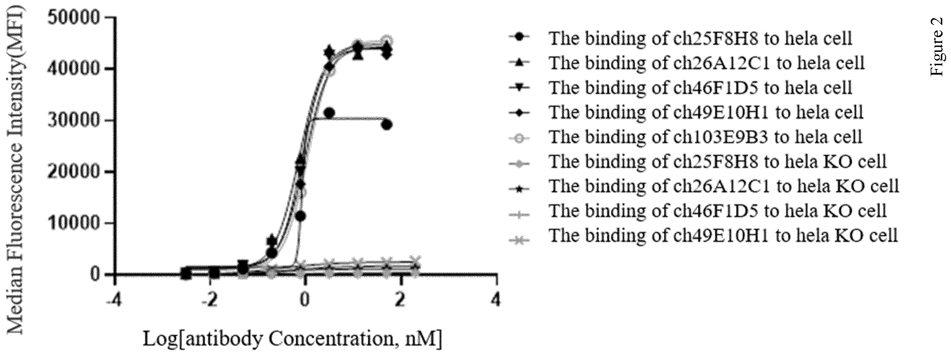

Figure 2, Binding curve of anti-L1CAM human-mouse chimeric antibody to HeLa cells and HeLa KO cells. Binding curve of anti-L1CAM human-mouse chimeric antibody to HeLa cells and HeLa KO cells

### Specific embodiment

Unless otherwise defined, technical and scientific terms used herein have the same meaning as commonly understood by the skilled person in the art. Furthermore, the terms and laboratory procedures related to protein and nucleic acid chemistry, molecular biology, cell and tissue culture, microbiology, and immunology used in this article are terms and routine procedures widely used in the corresponding fields. Meanwhile, in order to better understand the present invention, definitions and explanations of relevant terms are provided below.

The term "L1CAM" as used herein refers to any variant, isoform and species homologue of L1CAM. L1CAM is a transmembrane glycoprotein with a molecular weight between 200,000 and 220,000, also known as CD171. Structurally, the extracellular part of L1CAM consists of six immunoglobulin-like domains and five type III fibronectin repeats starting from the amino terminus of the protein. In addition, L1CAM also contains a transmembrane domain and an intracellular domain. domain. In certain embodiments, the L1CAM is human L1CAM. Exemplary sequences of human L1CAM proteins are disclosed in NCBI Ref Seq No. NP_001265045.1, NCBI Ref Seq No. NP_000416.1, NCBI Ref Seq No. NP_076493.1, and NCBI Ref Seq No. NP_001137435.1. Exemplary predicted sequences for the monkey L1 CAM protein are disclosed in NCBI Ref Seq No. XP_005594990.1, Ref Seq No. 1.

The term "antibody" as used herein refers to any immunoglobulin that binds to a specific antigen. A natural intact antibody contains two heavy (H) chains and two light (L) chains. Each heavy chain in mammals consists of a variable region (VH) and first, second, third and fourth (optionally) constant regions (CH1, CH2, CH3, CH4, respectively); mammalian light chains can be divided into lambda or kappa, and each light chain consists of a variable region (VL) and a constant region. The variable regions of the light and heavy chains determine antigen binding. The variable region of each chain usually contains three hypervariable regions, called complementarity determining regions (CDRs) (the light chain CDR includes LCDR1, LCDR2, and LCDR3, and the heavy chain CDR includes HCDR1, HCDR2, and HCDR3). The CDR boundaries of the antibodies and antigen-binding fragments disclosed in the present invention may be named or identified by Kabat nomenclature.

The term "monoclonal antibody" as used in the present invention refers to a population of identical antibodies, indicating that each individual antibody molecule in the monoclonal antibody population is the same as other antibody molecules. This characteristic is contrary to the characteristics of the polyclonal population of the antibody, the polyclonal population of the antibody comprises an antibody comprising a variety of different sequences.

The term "bispecific antibody" used in the present invention refers to an artificial antibody that has fragments derived from two different monoclonal antibodies and is capable of binding to two different epitopes. The two epitopes may be present on the same antigen, or they may be present on two different antigens.

The term "multispecific antibody" used in the present invention refers to an artificial antibody that has fragments derived from multiple different monoclonal antibodies and is capable of binding to multiple different epitopes. The multiple epitopes may be present on the same antigen, or they may be present on multiple different antigens.

The term "recombinant antibody" used in the present invention refers to antibodies produced by expression in engineered cell lines or engineering strains through recombinant genetic engineering technology.

The term "chimeric antibody" as used herein refers to an antibody molecule in which the antibody constant region or part thereof is replaced such that the antigen binding site (variable region, CDR or part thereof) is linked to the constant region of a different species.

The term "amino acid" as used in the present invention refers to organic compounds containing amino ((-NH₂) and carboxyl (-COOH) functional groups and side chains unique to each amino acid. Amino acid names are also used in the present invention in accordance with standards recognized in the art. represented by a one-letter or three-letter code.

"Treatment" or "therapy" for a disease, condition or condition as used in the present invention includes preventing or alleviating a disease, indication or condition, reducing the rate at which a disease, condition or condition occurs or develops, reducing the risk of developing a disease, indication or condition, preventing or delaying the development of symptoms associated with a disease, indication or condition, reducing or terminating symptoms associated with a disease, indication or condition, producing a complete or partial reversal of a disease, indication or condition, curing a disease, indication or condition, or a combination of those.

The term "diagnosis" as used herein refers to the identification of a pathological state, disease or condition, such as the identification of a cancer-related disease, or to the identification of a subject who may benefit from a particular treatment regimen.

### Example

The present invention will be further described by way of embodiment, but does not limit the present invention to the scope of the described embodiments.

### Embodiment 1:

### Production of monoclonal L1CAM antibodies

To generate L1CAM antibodies, Balb/c mice were immunized with recombinantly expressed human L1CAM antigen fragments. During the immune operation, blood was collected from the mice regularly to obtain serum samples and monitor the immune response. Mice with sufficient titers of anti-L1CAM antibodies were used for fusion experiments. Splenocytes and/or lymph node cells from immunized mice were isolated and fused with mouse myeloma cell lines. By performing ELISA analysis with the recombinant protein of the extracellular domain of human L1CAM, hybridomas that can produce L 1 CAM-specific antibodies were screened out using HeLa cells that highly express human L1CAM and HEK293 cells that highly express monkey L1CAM, and were subcloned to obtain stable of hybridoma clones. After subcloning, the monoclonal hybridoma cells are subjected to antibody sequencing for further analytical testing.

In the present invention, the mouse anti-L1CAM monoclonal antibody is represented by "hybridoma clone number". For example, the mouse anti-L1CAM monoclonal antibody derived from hybridoma clone 25F8H8 is represented by "25F8H8".

### Embodiment 2: Production of chimeric antibodies against L1CAM

Based on the results of monoclonal hybridoma cell antibody sequencing, human-mouse chimeric antibodies with CH1, CH2 and CH3 of human IgG1 as constant regions and VH and VL of murine anti-L1CAM monoclonal antibodies as variable regions were designed and recombinant expressed in mammalian cells and purified for further analysis and testing. In the present invention, the chimeric antibody number is indicated as "ch+ hybridoma clone number", for example, a chimeric antibody from hybridoma clone 25F8H8 is indicated as "ch25F8H8"

### Embodiment 3: The binding of mouse anti-L1CAM monoclonal antibody to HeLa cells

HeLa cells used in this example were used to measure the binding of mouse anti-L1CAM monoclonal antibodies to endogenously expressed human L1CAM on the cell surface. HeLa cells were cultured in DMEM complete medium (purchased from Corning) containing 10% FBS (purchased from Gibco) and 100 U/mL penicillin-streptomycin mixture (Thermofisher, 15140122). When the coverage of the cells in the culture container reached to 80-90% , using TrypLE (purchased from Thermofisher) to digest Hela cells from the culture container, and seed them in a 96-well plate (purchased from AXYGEN) at a density of 4×10⁶ cells/ml, with 100,000 cells per well (25 µL DMEM complete culture medium), then add 25 µL of diluted hybridoma supernatant containing antibodies to each well, mix gently, and incubate the 96-well plate at 4°C for 1 hour. Then add 150 µL of PBS buffer containing 2% FBS (purchased from Corning) to each well, centrifuge at 1400 rpm for 4 minutes, remove the supernatant, and repeat washing once. Then, 50 µL of 1:800 diluted Alexa Flour 647-labeled goat anti-mouse fluorescent secondary antibody (purchased from Jacksonimmuno) was added to each well, and the 96-well plate was incubated at 4°C for 0.5 hours. The cells were washed twice with PBS solution containing 2% FBS, and then resuspended in 120 µL of PBS. The fluorescence signal on the cell surface was detected using a flow cytometer (BD FACSCelesta) to obtain the median fluorescence intensity (MFI). The antibody-containing hybridoma supernatant was assayed using an anti-mouse ELISA assay to determine the concentration of mouse IgG in the supernatant. Finally, a dose-response curve was generated, and the half effective concentration (EC50) was calculated by nonlinear regression using GraphPad software.

**Table 1. The binding of mouse anti-L1CAM monoclonal antibody to HeLa cells**

| Antibody NO. | EC50 (nM) |
|---|---|
| 25F8H8 | 0.1927 |
| 26A12C1 | 0.2414 |
| 46F 1D5 | 0.1440 |
| 49E10H1 | 0.2333 |
| 103E9B3 | 0.4356 |

### Example 4: The binding of anti-L1CAM human-mouse chimeric antibodies to endogenously expressed human L1CAM on HeLa cells

This example utilizes HeLa cells and L1CAM-knockout HeLa cells (purchased from Abcam) to determine the binding of mouse anti-L1CAM monoclonal antibodies to endogenously expressed human L1CAM on the cell surface. L1CAM-knockout HeLa cells are referred to as HeLa KO cells in this invention.

HeLa cells are cultured in DMEM complete medium (purchased from Corning) containing 10% FBS (purchased from Gibco) and 100U/mL penicillin mixture (Thermofisher, 15140122). When the cell coverage in the culture container reaches 80-90%, TrypLE (purchased from Thermofisher) is used to digest the HeLa and HeLa KO cells from the culture container, and they are seeded in a 96-well plate (purchased from AXYGEN) at a density of 4x106 cells/ml, with 100,000 cells (25µL DMEM complete medium) per well. Then, the human-mouse chimeric antibodies are serially diluted in DMEM complete medium, with 25µL of the diluted chimeric antibody solution added to each well, gently mixed, and incubated at 4°C for 1 hour. Then, 150µL of PBS buffer containing 2% FBS (purchased from Corning) is added to each well, centrifuged at 1400 rpm for 4 minutes, the supernatant is removed, and the wells are washed once more. Then, 50µL of Alexa Flour 647-labeled goat anti-human fluorescent secondary antibody (purchased from Jacksonimmuno) diluted 1:800 is added to each well, and the 96-well plate is incubated at 4°C for 0.5 hours. The cells are washed twice with PBS solution containing 2% FBS, then resuspended in 120µL of PBS, and the cell surface fluorescence signal is detected using a flow cytometer (BD FACSCelesta), obtaining the median fluorescence intensity (MFI). A dose-response curve is generated, and the half-maximal effective concentration (EC50) is calculated using non-linear regression with GraphPad software. HeLa cells endogenously express high levels of L1CAM, and the expression level of L1CAM in HeLa KO cells is significantly reduced relative to wild-type HeLa due to the knockout of the L1CAM gene, allowing the binding ability of antibodies to L1CAM on the surface of HeLa cells to be determined by flow cytometry, and the specificity of the antibodies is assessed by detecting the fluorescence signal on the surface of HeLa KO cells.

| Table 2. The binding of anti-L1CAM human-mouse chimeric antibodies to HeLa cells and HeLa cells | HelaCell | | Hela KO Cell | |
|---|---|---|---|---|
| chimeric antibodies NO. | EC50(nM) | Maxium MFI | EC50(nM) | Maxium MFI |
| ch25F8H8 | 0.8331 | 29253 | 1.557 | 372 |
| ch26A12C1 | 0.7392 | 44705 | 0.319 | 1165 |
| ch46F 1D5 | 0.8452 | 43085 | 0.4688 | 1739 |
| ch49E10H1 | 0.9783 | 42858 | 0.3136 | 2640 |
| ch103E9B3 | 1.113 | 45397 | N/A | N/A |

| | | | | |
|---|---|---|---|---|
| Note: N/A represents data not available | | | | |

### Example 5: Production of humanized antibodies against L1CAM

Mouse anti-L1CAM monoclonal antibodies 46F1D5, 49E10H1, and 103E9B3 are selected as clones for humanization. The antibody sequences are aligned with human germline sequences to determine the best fit model. Based on homology with the original mouse antibody sequences, the most matching human germline sequences are selected as templates for humanization. The CDRs from the mouse antibody sequences are grafted onto the template along with residues that preserve the upper and central core structures of the antibody. Optimized mutations are introduced into the framework region to produce variants of the humanized heavy chain variable region and the humanized light chain variable region, which are mixed and matched to provide multiple humanized antibody clones. They are recombinantly expressed in mammalian cells and purified for further analytical testing.

### Example 6: Determination of humanized L1CAM antibody binding on the surface of HeLa cells

The experimental principle is as follows: HeLa cells express high levels of L1CAM protein on their surface. After incubating humanized L1CAM antibodies with the cells, the binding ability of the antibodies to L1CAM on the surface of HeLa cells can be determined by flow cytometry.

The experimental method is descripted as following:
Humanized L1CAM antibodies (Human IgG1-Kappa) are diluted in complete medium in a four-fold serial dilution, with seven concentration gradient points.

HeLa cells are cultured in DMEM complete medium (Corning, 10-013-CVR) containing 10% FBS (Gibco, 10099-141) and 100U/mL penicillin mixture (Thermofisher, 15140122). When the cell coverage in the culture container reaches 80-90%, TrypLE (Thermofisher, 12604-013) is used to digest the HeLa cells from the culture container, and they are seeded in a 96-well plate (AXYGEN, P-96-450V-C) at a density of 2.2 × 10⁶ cells/ml, with 100,000 cells (45µL complete medium) per well. Then, 5µL of the antibody dilution is added to each well, gently mixed, and the 96-well plate is incubated at 4°C for 1 hour. Then, 150µL of PBS solution containing 2% FBS (Corning, 21-040-CVC) is added to each well, centrifuged at 1400 rpm for 4 minutes, the supernatant is removed, and the wells are washed once more. Then, 50µL of Alexa Flour 647-labeled goat anti-human fluorescent secondary antibody (Jacksonimmuno, 109-605-088) diluted 1:800 is added to each well, and the 96-well plate is incubated at 4°C for 0.5 hours. The cells are washed twice with PBS solution containing 2% FBS, then resuspended in 120µL of PBS, and the cell surface fluorescence signal is detected using a flow cytometer (BD FACS Celesta), obtaining the median fluorescence intensity (MFI). A dose-response curve is generated, and the half-maximal effective concentration (EC50) is calculated using non-linear regression with GraphPad software.

**Table 3 Binding affinity to Hela cell of Humanized 26A12C1 antibody**

| Humanized antibody NO. | EC50(nM) |
|---|---|
| hz26A12C1H1L1 | N/A |
| hz26A12C1H1L2 | N/A |
| hz26A12C1H1L3 | N/A |
| hz26A12C1H2L1 | 24.69 |
| hz26A12C1H2L2 | 7.819 |
| hz26A12C1H2L3 | 0.8529 |
| hz26A12C1H3L1 | 2.988 |
| hz26A12C1H3L2 | 2.333 |
| hz26A12C1H3L3 | 0.838 |
| hz26A12C1H4L1 | 0.646 |
| hz26A12C1H4L2 | 0.5621 |
| hz26A12C1H4L3 | 0.7378 |

**Table 4 Binding affinity to Hela cell of Humanized 49E10H1 antibody**

| Humanized antibody NO. | EC50(nM) |
|---|---|
| hz49E10H1H4L5 | 2.15 |
| hz49E10H1H4L6 | 0.8533 |
| hz49E10H1H4L7 | 0.6764 |
| hz49E10H1H5L5 | 0.6326 |
| hz49E10H1H5L6 | 0.4042 |
| hz49E10H1H5L7 | 0.3767 |
| hz49E10H1H6L5 | 24.33 |
| hz49E10H1H6L6 | 1.03 |
| hz49E10H1H6L7 | 0.9962 |
| hz49E10H1H7L5 | 10.39 |
| hz49E10H1H7L6 | 1.051 |
| hz49E10H1H7L7 | 0.8516 |
| hz49E10H1H8L5 | 9.274 |
| hz49E10H1H8L6 | 1.06 |
| hz49E10H1H8L7 | 0.8204 |

**Table 5 Binding affinity to Hela cell of Humanized 103E9B3 antibody**

| Humanized antibody NO. | EC50(nM) |
|---|---|
| ch103E9B3 | 1.21 |
| hz103E9B3H5L4 | 6.845 |
| hz103E9B3H5L5 | 1.682 |
| hz103E9B3H5L6 | 1.926 |
| hz103E9B3H6L4 | 6.595 |
| hz103E9B3H6L5 | 1.483 |
| hz103E9B3H6L6 | 2.184 |
| hz103E9B3H7L4 | 5.323 |
| hz103E9B3H7L5 | 2.133 |
| hz103E9B3H7L6 | 2.034 |
| hz103E9B3H8L4 | 5.795 |
| hz103E9B3H8L5 | 1.54 |
| hz 103E9B3H8L6 | 1.997 |
| hz103E9B3H9L4 | 5.564 |
| hz103E9B3H9L5 | 1.964 |
| hz103E9B3H9L6 | 2.065 |

In the present invention, the humanized antibody number is indicated as "hz + hybridoma clone number + heavy chain number + light chain number", for example, the humanized antibody "hz46F1D5H1L2" represents a humanized antibody derived from the mouse anti-L1CAM monoclonal antibody 46F1D5, and the variable region heavy chain is VH1 (i.e., SEQ ID NO: 60), and the variable region light chain is VL2 (i.e., SEQ ID NO: 65).

In the present invention, the human-mouse chimeric antibody number is indicated as "ch+ hybridoma clone number", for example, the human-mouse chimeric antibody "ch46F1D5" represents the human-mouse chimeric antibody derived from the mouse anti-L1CAM monoclonal antibody 46F1D5, and the variable region heavy chain is the heavy chain variable region of the mouse monoclonal antibody 46F1D5, the variable region light chain is the light chain variable region of the mouse monoclonal antibody 46F1D5, and the constant region is the human IgG1 constant region.

### Antibody Sequence information:

**Table 6 Mouse monoclonal antibody complementary determinant amino acid sequence**

| Antibody NO. | Amino acid Sequence | | | SEQ ID NO. |
|---|---|---|---|---|
| 25F8H8 | HCDR | HCDR1 | TYWMH | 1 |
| | | HCDR2 | YINP S TGYTDYNQKFKD | 2 |
| | | HCDR3 | RQLGLPLFDY | 3 |
| | LCDR | LCDR1 | KASQDVGTAVA | 4 |
| | | LCDR2 | WASTRHT | 5 |
| | | LCDR3 | QQYSNYPLT | 6 |
| 26A12C 1 | HCDR | HCDR1 | GYTFTNYW | 7 |
| | | HCDR2 | INPSTYYS | 8 |
| | | HCDR3 | ARRQFGLPLFDY | 9 |
| | LCDR | LCDR1 | QDVGTA | 10 |
| | | LCDR2 | WAS | 11 |
| | | LCDR3 | QQYSNYPLT | 12 |
| 46F 1D5 | HCDR | HCDR1 | GYTFTRYW | 13 |
| | | HCDR2 | INPSTGYT | 14 |
| | | HCDR3 | ARRHLGLPLFDY | 15 |
| | LCDR | LCDR1 | QDVGTA | 16 |
| | | LCDR2 | WAS | 17 |
| | | LCDR3 | QQYSNYPLT | 18 |
| 49E10H 1 | HCDR | HCDR1 | GYSITSDYA | 19 |
| | | HCDR2 | ITYSGTT | 20 |
| | | HCDR3 | TLDHYGNYAYFDY | 21 |
| | LCDR | LCDR1 | SSVSF | 22 |
| | | LCDR2 | ASS | 23 |
| | | LCDR3 | QQWSSNPST | 24 |
| 49'E10H 1 | HCDR | HCDR1 | GGSISSDYA | 25 |
| | | HCDR2 | ITYSGTT | 26 |
| | | HCDR3 | ARDHYGNYAYFDY | 27 |
| | LCDR | LCDR1 | SSVSF | 28 |
| | | LCDR2 | ASS | 29 |
| | | LCDR3 | QQWSSNPST | 30 |
| 49"E10 H1 | HCDR | HCDR1 | GGSISSDYA | 31 |
| | | HCDR2 | ITYSGTT | 32 |
| | | HCDR3 | TLDHYGNYAYFDY | 33 |
| | LCDR | LCDR1 | SSVSF | 34 |
| | | LCDR2 | ASS | 35 |
| | | LCDR3 | QQWSSNPST | 36 |
| 103E9B3 | HCDR | HCDR1 | GYTFTSYT | 37 |
| | | HCDR2 | INPNYGGT | 38 |
| | | HCDR3 | AREGATGTWYFDV | 39 |
| | LCDR | LCDR1 | SSVSF | 40 |
| | | LCDR2 | LTS | 41 |
| | | LCDR3 | QQWRSNPPT | 42 |

**Table 7 Mouse monoclonal antibody amino acid sequence**

| Antibody | Amino acid sequence | | SEQ ID NO. |
|---|---|---|---|
| 25F8H8 | Variable full length of heavy chain (VH) | QVQLQQSGAELAKPGASVKMSCQASGYTF TTYWMHWVKQRPGQGLEWIGYINPSTGYT DYNQKFKDKATLTADKSSSTAYMQLSSLTS EDSAVYYCARRQLGLPLFDYWGQGTTLTV SS | 43 |
| | Variable full length of light chain (VL) | DIVMTQSHKFLSTSVGDRVSITCKASQDVG TAVAWYQHKPGQSPKLLIFWASTRHTGVPD RFTGSGSGTDFTLTISNVQSEDLADYFCQQY SNYPLTFGAGPKLELK | 44 |
| 26A12C 1 | Variable full length of heavy chain (VH) | MERHWIFLFLFSATAGVHSQVQLQQSGAEL AKPGASVKMSCKASGYTFTNYWMQWVKQ RPGQGLEWIGYINPSTYYSDYNQKFKDKAT LTADKSSSTAFMQLSSLTSEDSAVYYCARRQ FGLPLFDYWGQGTTLTLSS | 45 |
| | Variable full length of light chain (VL) | METHSQVFVYMLLWLSGVEGDIVMTQSHK FMSTSVGDRVSITCKASQDVGTAVAWYQQK PGQSPKLLIYWASTRHTGVPDRFTGSGSGT DFTLTISNVQSEDLTDYFCQQYSNYPLTFGA GTELELK | 46 |
| 46F 1D5 | Variable full length of heavy chain (VH) | MERHWIFLFLFSVTAGVYSQVQVQQSGAEL AKPGASMKMSCKASGYTFTRYWMQWVK QRPGQGLEWIGYINPSTGYTDYNQNFKDK ATLTADKSSSTAYMQLSSLTSEDSAVYYCAR RHLGLPLFDYWGQGTTLTVSS | 47 |
| | Variable full length of light chain (VL) | METHSQVFVYMLLWLSGVEGDIVMTQSHK FMSTSLGDRVSITCKASQDVGTAVAWYQQK PGQSPKLLIYWASTRHTGVPDRFTGSGSGT DFTLTISNVQSEDLAEYFCQQYSNYPLTFGA GTKLDLK | 48 |
| 49E10H | Variable | MRVLILLWLFTAFPGILSDVQLQESGPGLVK | 49 |
| 1 | full length of heavy chain (VH) | PSQSLSLTCTVTGYSITSDYAWNWIRQFPGN RLEWMGYITYSGTTTYNPSLKSRISITRDTS KNQFFLQLNSVTTADTATYYCTLDHYGNYA YFDYWGQGTTLTVSS | |
| | Variable full length of light chain (VL) | MDFQVQIFSFLLISASVIMSRGQIVLSQSPAI LSASPGEKVTMTCRASSSVSFMHWYQQKP RSSPKPWIYASSNLVSGVPTRFSGSGSGTSY SLTISRVEAEDAATYYCQQWSSNPSTFGAGT KLELK | 50 |
| 103E9B3 | Variable full length of heavy chain (VH) | MGWSWIFLFLLSGTAGVLSEVQLQQSGPEL LKPGASVKISCKASGYTFTSYTMHWVKQS HGKSLEWIGDINPNYGGTSYNEKFKDKATL TVDKSSSTAYMELRSLTSEDSAVYYCAREG ATGTWYFDVWGTGTTVTVSS | 51 |
| | Variable full length of light chain (VL) | MDFQVQIFSFLLMSASVIMSRGQIVLTQSPA LMSASPGEKVTMTCSASSSVSFMFWYQQK PRSSPKPWIYLTSNLASGVPARFSGSGSGTS YSLTISSMEAEDAATYYCQQWRSNPPTFGA GTKLELK | 52 |

**Table 8 Humanized monoclonal antibody variable region amino acid sequence**

| Antibody NO. | SEQ ID NO: | | Amino acid sequence |
|---|---|---|---|
| hz26A12C1H1 L1 | VH | 53 | QVQLVQSGAEVKKPGSSVKVSCKASGYT FTNYWISWVRQAPGQGLEWMGGINPSTY YSNYAQKFQGRVTITADESTSTAYMELSSL RSEDTAVYYCARRQFGLPLFDYWGQGTT VTVSS |
| | VL | 57 | DIQMTQSPSSLSASVGDRVTITCRASQDV GTALAWYQQKPGKVPKLLIYWASTLQSG VPSRFSGSGSGTDFTLTISSLQPEDVATYY CQQYSNYPLTFGGGTKVEIK |
| hz26A12C1H1 L2 | VH | 53 | QVQLVQSGAEVKKPGSSVKVSCKASGYT FTNYWISWVRQAPGQGLEWMGGINPSTY YSNYAQKFQGRVTITADESTSTAYMELSSL RSEDTAVYYCARRQFGLPLFDYWGQGTT VTVSS |
| | VL | 58 | DIQMTQSPSSLSASVGDRVTITCRASQDV GTAVAWYQQKPGKVPKLLIYWASTLHSG VPSRFSGSGSGTDFTLTISSLQPEDVATYY CQQYSNYPLTFGGGTKVEIK |
| hz26A12C1H1 L3 | VH | 53 | QVQLVQSGAEVKKPGSSVKVSCKASGYT FTNYWISWVRQAPGQGLEWMGGINPSTY YSNYAQKFQGRVTITADESTSTAYMELSSL RSEDTAVYYCARRQFGLPLFDYWGQGTT VTVSS |
| | VL | 59 | DIQMTQSPSSLSASVGDRVTITCRASQDV GTAVAWYQQKPGKVPKLLIYWASTLHSG VPSRFSGSGSGTDFTLTISSLQPEDVATYFC QQYSNYPLTFGGGTKVEIK |
| hz26A12C1H2 L1 | VH | 54 | QVQLVQSGAEVKKPGSSVKVSCKASGYT FTNYWIQWVRQAPGQGLEWMGYINPST YYSDYAQKFQGRVTITADESTSTAYMELS SLRSEDTAVYYCARRQFGLPLFDYWGQG TTVTVSS |
| | VL | 57 | DIQMTQSPSSLSASVGDRVTITCRASQDV GTALAWYQQKPGKVPKLLIYWASTLQSG VPSRFSGSGSGTDFTLTISSLQPEDVATYY CQQYSNYPLTFGGGTKVEIK |
| hz26A12C1H2 L2 | VH | 54 | QVQLVQSGAEVKKPGSSVKVSCKASGYT FTNYWIQWVRQAPGQGLEWMGYINPST YYSDYAQKFQGRVTITADESTSTAYMELS SLRSEDTAVYYCARRQFGLPLFDYWGQG TTVTVSS |
| | VL | 58 | DIQMTQSPSSLSASVGDRVTITCRASQDV GTAVAWYQQKPGKVPKLLIYWASTLHSG VPSRFSGSGSGTDFTLTISSLQPEDVATYY CQQYSNYPLTFGGGTKVEIK |
| hz26A12C1H2 L3 | VH | 54 | QVQLVQSGAEVKKPGSSVKVSCKASGYT FTNYWIQWVRQAPGQGLEWMGYINPST YYSDYAQKFQGRVTITADESTSTAYMELS SLRSEDTAVYYCARRQFGLPLFDYWGQG TTVTVSS |
| | VL | 59 | DIQMTQSPSSLSASVGDRVTITCRASQDV GTAVAWYQQKPGKVPKLLIYWASTLHSG VPSRFSGSGSGTDFTLTISSLQPEDVATYFC QQYSNYPLTFGGGTKVEIK |
| hz26A12C1H3 L1 | VH | 55 | QVQLVQSGAEVKKPGSSVKVSCKASGYT FTNYWIQWVRQAPGQGLEWIGYINPSTY YSDYAQKFQGRVTLTADESTSTAYMELSS LRSEDTAVYYCARRQFGLPLFDYWGQGT TVTVSS |
| | VL | 57 | DIQMTQSPSSLSASVGDRVTITCRASQDV GTALAWYQQKPGKVPKLLIYWASTLQSG VPSRFSGSGSGTDFTLTISSLQPEDVATYY CQQYSNYPLTFGGGTKVEIK |
| hz26A12C1H3 L2 | VH | 55 | QVQLVQSGAEVKKPGSSVKVSCKASGYT FTNYWIQWVRQAPGQGLEWIGYINPSTY YSDYAQKFQGRVTLTADESTSTAYMELSS LRSEDTAVYYCARRQFGLPLFDYWGQGT TVTVSS |
| | VL | 58 | DIQMTQSPSSLSASVGDRVTITCRASQDV GTAVAWYQQKPGKVPKLLIYWASTLHSG VPSRFSGSGSGTDFTLTISSLQPEDVATYY CQQYSNYPLTFGGGTKVEIK |
| hz26A12C1H3 L3 | VH | 55 | QVQLVQSGAEVKKPGSSVKVSCKASGYT FTNYWIQWVRQAPGQGLEWIGYINPSTY YSDYAQKFQGRVTLTADESTSTAYMELSS LRSEDTAVYYCARRQFGLPLFDYWGQGT TVTVSS |
| | VL | 59 | DIQMTQSPSSLSASVGDRVTITCRASQDV GTAVAWYQQKPGKVPKLLIYWASTLHSG VPSRFSGSGSGTDFTLTISSLQPEDVATYFC QQYSNYPLTFGGGTKVEIK |
| hz26A12C1H4 L1 | VH | 56 | QVQLVQSGAEVKKPGSSVKVSCKASGYT FTNYWIQWVRQAPGQGLEWIGYINPSTY YSDYNQKFQGRVTLTADKSTSTAYMELSS LRSEDTAVYYCARRQFGLPLFDYWGQGT TVTVSS |
| | VL | 57 | DIQMTQSPSSLSASVGDRVTITCRASQDV GTALAWYQQKPGKVPKLLIYWASTLQSG VPSRFSGSGSGTDFTLTISSLQPEDVATYY CQQYSNYPLTFGGGTKVEIK |
| hz26A12C1H4 L2 | VH | 56 | QVQLVQSGAEVKKPGSSVKVSCKASGYT FTNYWIQWVRQAPGQGLEWIGYINPSTY YSDYNQKFQGRVTLTADKSTSTAYMELSS LRSEDTAVYYCARRQFGLPLFDYWGQGT TVTVSS |
| | VL | 58 | DIQMTQSPSSLSASVGDRVTITCRASQDV GTAVAWYQQKPGKVPKLLIYWASTLHSG VPSRFSGSGSGTDFTLTISSLQPEDVATYY CQQYSNYPLTFGGGTKVEIK |
| hz26A12C1H4 L3 | VH | 56 | QVQLVQSGAEVKKPGSSVKVSCKASGYT FTNYWIQWVRQAPGQGLEWIGYINPSTY YSDYNQKFQGRVTLTADKSTSTAYMELSS LRSEDTAVYYCARRQFGLPLFDYWGQGT TVTVSS |
| | VL | 59 | DIQMTQSPSSLSASVGDRVTITCRASQDV GTAVAWYQQKPGKVPKLLIYWASTLHSG VPSRFSGSGSGTDFTLTISSLQPEDVATYFC QQYSNYPLTFGGGTKVEIK |
| hz46F1D5H1L 1 | VH | 60 | QVQLVQSGAEVKKPGSSVKVSCKASGYT FTRYWISWVRQAPGQGLEWMGGINPSTG YTNYAQKFQGRVTITADESTSTAYMELSS LRSEDTAVYYCARRHLGLPLFDYWGQGT TVTVSS |
| | VL | 64 | DIQMTQSPSSVSASVGDRVTITCRASQDV GTALAWYQQKPGKAPKLLIYWASSLQSG VPSRFSGSGSGTDFTLTISSLQPEDFATYYC QQYSNYPLTFGGGTKVEIK |
| hz46F1D5H1L 2 | VH | 60 | QVQLVQSGAEVKKPGSSVKVSCKASGYT FTRYWISWVRQAPGQGLEWMGGINPSTG YTNYAQKFQGRVTITADESTSTAYMELSS LRSEDTAVYYCARRHLGLPLFDYWGQGT TVTVSS |
| | VL | 65 | DIQMTQSPSSVSASVGDRVTITCRASQDV GTALAWYQQKPGKAPKLLIYWASSLHSG VPSRFSGSGSGTDFTLTISSLQPEDFATYYC QQYSNYPLTFGGGTKVEIK |
| hz46F1D5H1L 3 | VH | 60 | QVQLVQSGAEVKKPGSSVKVSCKASGYT FTRYWISWVRQAPGQGLEWMGGINPSTG YTNYAQKFQGRVTITADESTSTAYMELSS LRSEDTAVYYCARRHLGLPLFDYWGQGT TVTVSS |
| | VL | 66 | DIQMTQSPSSVSASVGDRVTITCRASQDV GTAVAWYQQKPGKAPKLLIYWASSLHSG VPSRFSGSGSGTDFTLTISSLQPEDFATYFC QQYSNYPLTFGGGTKVEIK |
| hz46F1D5H2L 1 | VH | 61 | QVQLVQSGAEVKKPGSSVKVSCKASGYT FTRYWIQWVRQAPGQGLEWMGYINPSTG YTNYAQKFQGRVTITADESTSTAYMELSS LRSEDTAVYYCARRHLGLPLFDYWGQGT TVTVSS |
| | VL | 64 | DIQMTQSPSSVSASVGDRVTITCRASQDV GTALAWYQQKPGKAPKLLIYWASSLQSG VPSRFSGSGSGTDFTLTISSLQPEDFATYYC QQYSNYPLTFGGGTKVEIK |
| hz46F1D5 H2L2 | VH | 61 | QVQLVQSGAEVKKPGSSVKVSCKASGYT FTRYWIQWVRQAPGQGLEWMGYINPSTG YTNYAQKFQGRVTITADESTSTAYMELSS LRSEDTAVYYCARRHLGLPLFDYWGQGT TVTVSS |
| | VL | 65 | DIQMTQSPSSVSASVGDRVTITCRASQDV GTALAWYQQKPGKAPKLLIYWASSLHSG VPSRFSGSGSGTDFTLTISSLQPEDFATYYC QQYSNYPLTFGGGTKVEIK |
| hz46F1D5 H2L3 | VH | 61 | QVQLVQSGAEVKKPGSSVKVSCKASGYT FTRYWIQWVRQAPGQGLEWMGYINPSTG YTNYAQKFQGRVTITADESTSTAYMELSS LRSEDTAVYYCARRHLGLPLFDYWGQGT TVTVSS |
| | VL | 66 | DIQMTQSPSSVSASVGDRVTITCRASQDV GTAVAWYQQKPGKAPKLLIYWASSLHSG VPSRFSGSGSGTDFTLTISSLQPEDFATYFC QQYSNYPLTFGGGTKVEIK |
| hz46F1D5H3L 1 | VH | 62 | QVQLVQSGAEVKKPGSSVKVSCKASGYT FTRYWIQWVRQAPGQGLEWMGYINPSTG YTDYNQKFQGRVTITADESTSTAYMELSS LRSEDTAVYYCARRHLGLPLFDYWGQGT TVTVSS |
| | VL | 64 | DIQMTQSPSSVSASVGDRVTITCRASQDV GTALAWYQQKPGKAPKLLIYWASSLQSG VPSRFSGSGSGTDFTLTISSLQPEDFATYYC QQYSNYPLTFGGGTKVEIK |
| hz46F1D5 H3L2 | VH | 62 | QVQLVQSGAEVKKPGSSVKVSCKASGYT FTRYWIQWVRQAPGQGLEWMGYINPSTG YTDYNQKFQGRVTITADESTSTAYMELSS LRSEDTAVYYCARRHLGLPLFDYWGQGT TVTVSS |
| | VL | 65 | DIQMTQSPSSVSASVGDRVTITCRASQDV GTALAWYQQKPGKAPKLLIYWASSLHSG VPSRFSGSGSGTDFTLTISSLQPEDFATYYC QQYSNYPLTFGGGTKVEIK |
| hz46F1D5 H3L3 | VH | 62 | QVQLVQSGAEVKKPGSSVKVSCKASGYT FTRYWIQWVRQAPGQGLEWMGYINPSTG YTDYNQKFQGRVTITADESTSTAYMELSS LRSEDTAVYYCARRHLGLPLFDYWGQGT TVTVSS |
| | VL | 66 | DIQMTQSPSSVSASVGDRVTITCRASQDV GTAVAWYQQKPGKAPKLLIYWASSLHSG VPSRFSGSGSGTDFTLTISSLQPEDFATYFC QQYSNYPLTFGGGTKVEIK |
| hz46F1D5H4L 1 | VH | 63 | QVQLVQSGAEVKKPGSSVKVSCKASGYT FTRYWIQWVRQAPGQGLEWMGYINPSTG YTDYNQKFQGRVTITADKSTSTAYMELSS LRSEDTAVYYCARRHLGLPLFDYWGQGT TVTVSS |
| | VL | 64 | DIQMTQSPSSVSASVGDRVTITCRASQDV GTALAWYQQKPGKAPKLLIYWASSLQSG VPSRFSGSGSGTDFTLTISSLQPEDFATYYC QQYSNYPLTFGGGTKVEIK |
| hz46F1D5 H4L2 | VH | 63 | QVQLVQSGAEVKKPGSSVKVSCKASGYT FTRYWIQWVRQAPGQGLEWMGYINPSTG YTDYNQKFQGRVTITADKSTSTAYMELSS LRSEDTAVYYCARRHLGLPLFDYWGQGT TVTVSS |
| | VL | 65 | DIQMTQSPSSVSASVGDRVTITCRASQDV GTALAWYQQKPGKAPKLLIYWASSLHSG VPSRFSGSGSGTDFTLTISSLQPEDFATYYC QQYSNYPLTFGGGTKVEIK |
| hz46F1D5 H4L3 | VH | 63 | QVQLVQSGAEVKKPGSSVKVSCKASGYT FTRYWIQWVRQAPGQGLEWMGYINPSTG YTDYNQKFQGRVTITADKSTSTAYMELSS LRSEDTAVYYCARRHLGLPLFDYWGQGT TVTVSS |
| | VL | 66 | DIQMTQSPSSVSASVGDRVTITCRASQDV GTAVAWYQQKPGKAPKLLIYWASSLHSG VPSRFSGSGSGTDFTLTISSLQPEDFATYFC QQYSNYPLTFGGGTKVEIK |
| hz49E10H1H1 L1 | VH | 67 | QVQLQESGPGLVKPSQTLSLTCTVSGYSIT SDYAWSWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H1 L2 | VH | 67 | QVQLQESGPGLVKPSQTLSLTCTVSGYSIT SDYAWSWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H1 L3 | VH | 67 | QVQLQESGPGLVKPSQTLSLTCTVSGYSIT SDYAWSWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H1 L4 | VH | 67 | QVQLQESGPGLVKPSQTLSLTCTVSGYSIT SDYAWSWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H1 L5 | VH | 67 | QVQLQESGPGLVKPSQTLSLTCTVSGYSIT SDYAWSWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H1 L6 | VH | 67 | QVQLQESGPGLVKPSQTLSLTCTVSGYSIT SDYAWSWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H1 L7 | VH | 67 | QVQLQESGPGLVKPSQTLSLTCTVSGYSIT SDYAWSWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz49E10H1H2 L1 | VH | 68 | DVQLQESGPGLVKPSQTLSLTCTVSGYSIT SDYAWNWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H2 L2 | VH | 68 | DVQLQESGPGLVKPSQTLSLTCTVSGYSIT SDYAWNWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H2 L3 | VH | 68 | DVQLQESGPGLVKPSQTLSLTCTVSGYSIT SDYAWNWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H2 L4 | VH | 68 | DVQLQESGPGLVKPSQTLSLTCTVSGYSIT SDYAWNWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H2 L5 | VH | 68 | DVQLQESGPGLVKPSQTLSLTCTVSGYSIT SDYAWNWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H2 L6 | VH | 68 | DVQLQESGPGLVKPSQTLSLTCTVSGYSIT SDYAWNWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H2 L7 | VH | 68 | DVQLQESGPGLVKPSQTLSLTCTVSGYSIT SDYAWNWIRQPPGKGLEWIGYITYSGTTY YNPSLKSRVTISVDTSKNQFSLKLSSVTAA DTAVYYCTLDHYGNYAYFDYWGQGTTV TVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz49E10H1H3 L1 | VH | 69 | DVQLQESGPGLVKPSQTLSLTCTVSGYSIT SDYAWNWIRQPPGKGLEWMGYITYSGTT YYNPSLKSRVTISRDTSKNQFSLKLSSVTA ADTAVYYCTLDHYGNYAYFDYWGQGTT VTVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H3 L2 | VH | 69 | DVQLQESGPGLVKPSQTLSLTCTVSGYSIT SDYAWNWIRQPPGKGLEWMGYITYSGTT YYNPSLKSRVTISRDTSKNQFSLKLSSVTA ADTAVYYCTLDHYGNYAYFDYWGQGTT VTVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H3 L3 | VH | 69 | DVQLQESGPGLVKPSQTLSLTCTVSGYSIT SDYAWNWIRQPPGKGLEWMGYITYSGTT YYNPSLKSRVTISRDTSKNQFSLKLSSVTA ADTAVYYCTLDHYGNYAYFDYWGQGTT VTVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H3 L4 | VH | 69 | DVQLQESGPGLVKPSQTLSLTCTVSGYSIT SDYAWNWIRQPPGKGLEWMGYITYSGTT YYNPSLKSRVTISRDTSKNQFSLKLSSVTA ADTAVYYCTLDHYGNYAYFDYWGQGTT VTVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H3 L5 | VH | 69 | DVQLQESGPGLVKPSQTLSLTCTVSGYSIT SDYAWNWIRQPPGKGLEWMGYITYSGTT YYNPSLKSRVTISRDTSKNQFSLKLSSVTA ADTAVYYCTLDHYGNYAYFDYWGQGTT VTVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H3 L6 | VH | 69 | DVQLQESGPGLVKPSQTLSLTCTVSGYSIT SDYAWNWIRQPPGKGLEWMGYITYSGTT YYNPSLKSRVTISRDTSKNQFSLKLSSVTA ADTAVYYCTLDHYGNYAYFDYWGQGTT VTVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H3 L7 | VH | 69 | DVQLQESGPGLVKPSQTLSLTCTVSGYSIT SDYAWNWIRQPPGKGLEWMGYITYSGTT YYNPSLKSRVTISRDTSKNQFSLKLSSVTA ADTAVYYCTLDHYGNYAYFDYWGQGTT VTVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz49E10H1H4 L1 | VH | 70 | QVQLQESGPGLVKPSQTLSLTCTVSGGSIS SDYAWNWIRQPPGKGLEWIGYITYSGTTT YNPSLKSRVTISVDTSKNQFSLKLSSVTAA DTAVYYCARDHYGNYAYFDYWGQGTTV TVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H4 L2 | VH | 70 | QVQLQESGPGLVKPSQTLSLTCTVSGGSIS SDYAWNWIRQPPGKGLEWIGYITYSGTTT YNPSLKSRVTISVDTSKNQFSLKLSSVTAA DTAVYYCARDHYGNYAYFDYWGQGTTV TVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H4 L3 | VH | 70 | QVQLQESGPGLVKPSQTLSLTCTVSGGSIS SDYAWNWIRQPPGKGLEWIGYITYSGTTT YNPSLKSRVTISVDTSKNQFSLKLSSVTAA DTAVYYCARDHYGNYAYFDYWGQGTTV TVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H4 L4 | VH | 70 | QVQLQESGPGLVKPSQTLSLTCTVSGGSIS SDYAWNWIRQPPGKGLEWIGYITYSGTTT YNPSLKSRVTISVDTSKNQFSLKLSSVTAA DTAVYYCARDHYGNYAYFDYWGQGTTV TVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H4 L5 | VH | 70 | QVQLQESGPGLVKPSQTLSLTCTVSGGSIS SDYAWNWIRQPPGKGLEWIGYITYSGTTT YNPSLKSRVTISVDTSKNQFSLKLSSVTAA DTAVYYCARDHYGNYAYFDYWGQGTTV TVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H4 L6 | VH | 70 | QVQLQESGPGLVKPSQTLSLTCTVSGGSIS SDYAWNWIRQPPGKGLEWIGYITYSGTTT YNPSLKSRVTISVDTSKNQFSLKLSSVTAA DTAVYYCARDHYGNYAYFDYWGQGTTV TVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H4 L7 | VH | 70 | QVQLQESGPGLVKPSQTLSLTCTVSGGSIS SDYAWNWIRQPPGKGLEWIGYITYSGTTT YNPSLKSRVTISVDTSKNQFSLKLSSVTAA DTAVYYCARDHYGNYAYFDYWGQGTTV TVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz49E10H1H5 L1 | VH | 71 | DVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCARDHYGNYAYFDYWGQGTT VTVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H5 L2 | VH | 71 | DVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCARDHYGNYAYFDYWGQGTT VTVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H5 L3 | VH | 71 | DVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCARDHYGNYAYFDYWGQGTT VTVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H5 L4 | VH | 71 | DVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCARDHYGNYAYFDYWGQGTT VTVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H5 L5 | VH | 71 | DVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCARDHYGNYAYFDYWGQGTT VTVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H5 L6 | VH | 71 | DVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCARDHYGNYAYFDYWGQGTT VTVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H5 L7 | VH | 71 | DVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCARDHYGNYAYFDYWGQGTT VTVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz49E10H1H6 L1 | VH | 72 | DVQLQESGPGLVKPSQTLSLTCTVSGGSIS SDYAWNWIRQPPGKGLEWIGYITYSGTTT YNPSLKSRVTISRDTSKNQFSLKLSSVTAA DTAVYYCTLDHYGNYAYFDYWGQGTTV TVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H6 L2 | VH | 72 | DVQLQESGPGLVKPSQTLSLTCTVSGGSIS SDYAWNWIRQPPGKGLEWIGYITYSGTTT YNPSLKSRVTISRDTSKNQFSLKLSSVTAA DTAVYYCTLDHYGNYAYFDYWGQGTTV TVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H6 L3 | VH | 72 | DVQLQESGPGLVKPSQTLSLTCTVSGGSIS SDYAWNWIRQPPGKGLEWIGYITYSGTTT YNPSLKSRVTISRDTSKNQFSLKLSSVTAA DTAVYYCTLDHYGNYAYFDYWGQGTTV TVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H6 L4 | VH | 72 | DVQLQESGPGLVKPSQTLSLTCTVSGGSIS SDYAWNWIRQPPGKGLEWIGYITYSGTTT YNPSLKSRVTISRDTSKNQFSLKLSSVTAA DTAVYYCTLDHYGNYAYFDYWGQGTTV TVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H6 L5 | VH | 72 | DVQLQESGPGLVKPSQTLSLTCTVSGGSIS SDYAWNWIRQPPGKGLEWIGYITYSGTTT YNPSLKSRVTISRDTSKNQFSLKLSSVTAA DTAVYYCTLDHYGNYAYFDYWGQGTTV TVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H6 L6 | VH | 72 | DVQLQESGPGLVKPSQTLSLTCTVSGGSIS SDYAWNWIRQPPGKGLEWIGYITYSGTTT YNPSLKSRVTISRDTSKNQFSLKLSSVTAA DTAVYYCTLDHYGNYAYFDYWGQGTTV TVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H6 L7 | VH | 72 | DVQLQESGPGLVKPSQTLSLTCTVSGGSIS SDYAWNWIRQPPGKGLEWIGYITYSGTTT YNPSLKSRVTISRDTSKNQFSLKLSSVTAA DTAVYYCTLDHYGNYAYFDYWGQGTTV TVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz49E10H1H7 L1 | VH | 73 | QVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCTLDHYGNYAYFDYWGQGTT VTVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H7 L2 | VH | 73 | QVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCTLDHYGNYAYFDYWGQGTT VTVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H7 L3 | VH | 73 | QVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCTLDHYGNYAYFDYWGQGTT VTVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H7 L4 | VH | 73 | QVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCTLDHYGNYAYFDYWGQGTT VTVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H7 L5 | VH | 73 | QVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCTLDHYGNYAYFDYWGQGTT VTVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H7 L6 | VH | 73 | QVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCTLDHYGNYAYFDYWGQGTT VTVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H7 L7 | VH | 73 | QVQLQESGPGLVKPSQTLSLTCTVSGGSIS SDYAWNWIRQPPGKGLEWMGYITYSGTT TYNPSLKSRITISRDTSKNQFSLKLSSVTA ADTAVYYCTLDHYGNYAYFDYWGQGTT VTVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz49E10H1H8 L1 | VH | 74 | DVQLQESGPGLVKPSQTLSLTCTVSGGSIS SDYAWNWIRQPPGNGLEWMGYITYSGTT TYNPSLKSRITISRDTSKNQFSLKLSSVTA ADTAVYYCTLDHYGNYAYFDYWGQGTT VTVSS |
| | VL | 75 | DIQMTQSPSSLSASVGDRVTITCRASSSVS FLNWYQQKPGKAPKLLIYASSSLQSGVPS RFSGSGSGTDFTLTISSLQPEDFATYYCQQ WSSNPSTFGGGTKVEIK |
| hz49E10H1H8 L2 | VH | 74 | DVQLQESGPGLVKPSQTLSLTCTVSGGSIS SDYAWNWIRQPPGNGLEWMGYITYSGTT TYNPSLKSRITISRDTSKNQFSLKLSSVTA ADTAVYYCTLDHYGNYAYFDYWGQGTT VTVSS |
| | VL | 76 | DIQMTQSPSSLSASVGDRVTITCRASSSVS FLHWYQQKPGKAPKLLIYASSSLQSGVPS RFSGSGSGTDFTLTISSLQPEDFATYYCQQ WSSNPSTFGGGTKVEIK |
| hz49E10H1H8 L3 | VH | 74 | DVQLQESGPGLVKPSQTLSLTCTVSGGSIS SDYAWNWIRQPPGNGLEWMGYITYSGTT TYNPSLKSRITISRDTSKNQFSLKLSSVTA ADTAVYYCTLDHYGNYAYFDYWGQGTT VTVSS |
| | VL | 77 | DIQMTQSPSSLSASVGDRVTITCRASSSVS FMHWYQQKPGKAPKLLIYASSSLQSGVPS RFSGSGSGTDFTLTISSLQPEDFATYYCQQ WSSNPSTFGGGTKVEIK |
| hz49E10H1H8 L4 | VH | 74 | DVQLQESGPGLVKPSQTLSLTCTVSGGSIS SDYAWNWIRQPPGNGLEWMGYITYSGTT TYNPSLKSRITISRDTSKNQFSLKLSSVTA ADTAVYYCTLDHYGNYAYFDYWGQGTT VTVSS |
| | VL | 78 | DIQMTQSPSSLSASVGDRVTITCRASSSVS FMHWYQQKPGKAPKPLIYASSSLQSGVPS RFSGSGSGTDFTLTISSLQPEDFATYYCQQ WSSNPSTFGGGTKVEIK |
| hz49E10H1H8 L5 | VH | 74 | DVQLQESGPGLVKPSQTLSLTCTVSGGSIS SDYAWNWIRQPPGNGLEWMGYITYSGTT TYNPSLKSRITISRDTSKNQFSLKLSSVTA ADTAVYYCTLDHYGNYAYFDYWGQGTT VTVSS |
| | VL | 79 | DIQMTQSPSSLSASVGDRVTITCRASSSVS FMHWYQQKPGKAPKLLIYASSNLVSGVPS RFSGSGSGTDFTLTISSLQPEDFATYYCQQ WSSNPSTFGGGTKVEIK |
| hz49E10H1H8 L6 | VH | 74 | DVQLQESGPGLVKPSQTLSLTCTVSGGSIS SDYAWNWIRQPPGNGLEWMGYITYSGTT TYNPSLKSRITISRDTSKNQFSLKLSSVTA ADTAVYYCTLDHYGNYAYFDYWGQGTT VTVSS |
| | VL | 80 | DIQMTQSPSSLSASVGDRVTITCRASSSVS FMHWYQQKPGKAPKPWIYASSNLVSGVP SRFSGSGSGTDFTLTISSLQPEDFATYYCQ QWSSNPSTFGGGTKVEIK |
| hz49E10H1H8 L7 | VH | 74 | DVQLQESGPGLVKPSQTLSLTCTVSGGSIS SDYAWNWIRQPPGNGLEWMGYITYSGTT TYNPSLKSRITISRDTSKNQFSLKLSSVTA ADTAVYYCTLDHYGNYAYFDYWGQGTT VTVSS |
| | VL | 81 | QIQMTQSPSSLSASVGDRVTITCRASSSVS FMHWYQQKPGKAPKPWIYASSNLVSGVP SRFSGSGSGTDYTLTISSLQPEDFATYYCQ QWSSNPSTFGGGTKVEIK |
| hz103E9B3H1 L1 | VH | 82 | EVQLVQSGAEVKKPGASVKVSCKASGYT FTSYTMHWVRQAPGQGLEWMGIINPNYG GTSYAQKFQGRVTMTRDTSTSTVYMELS SLRSEDTAVYYCAREGATGTWYFDVWGQ GTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H1 L2 | VH | 82 | EVQLVQSGAEVKKPGASVKVSCKASGYT FTSYTMHWVRQAPGQGLEWMGIINPNYG GTSYAQKFQGRVTMTRDTSTSTVYMELS SLRSEDTAVYYCAREGATGTWYFDVWGQ GTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H1 L3 | VH | 82 | EVQLVQSGAEVKKPGASVKVSCKASGYT FTSYTMHWVRQAPGQGLEWMGIINPNYG GTSYAQKFQGRVTMTRDTSTSTVYMELS SLRSEDTAVYYCAREGATGTWYFDVWGQ GTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H1 L4 | VH | 82 | EVQLVQSGAEVKKPGASVKVSCKASGYT FTSYTMHWVRQAPGQGLEWMGIINPNYG GTSYAQKFQGRVTMTRDTSTSTVYMELS SLRSEDTAVYYCAREGATGTWYFDVWGQ GTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H1 L5 | VH | 82 | EVQLVQSGAEVKKPGASVKVSCKASGYT FTSYTMHWVRQAPGQGLEWMGIINPNYG GTSYAQKFQGRVTMTRDTSTSTVYMELS SLRSEDTAVYYCAREGATGTWYFDVWGQ GTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H1 L6 | VH | 82 | EVQLVQSGAEVKKPGASVKVSCKASGYT FTSYTMHWVRQAPGQGLEWMGIINPNYG GTSYAQKFQGRVTMTRDTSTSTVYMELS SLRSEDTAVYYCAREGATGTWYFDVWGQ GTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H2 L1 | VH | 83 | EVQLVQSGAEVKKPGASVKVSCKASGYT FTSYTMHWVRQAPGQGLEWMGDINPNY GGTSYAQKFQGRVTMTRDTSTSTVYMEL SSLRSEDTAVYYCAREGATGTWYFDVWG QGTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H2 L2 | VH | 83 | EVQLVQSGAEVKKPGASVKVSCKASGYT FTSYTMHWVRQAPGQGLEWMGDINPNY GGTSYAQKFQGRVTMTRDTSTSTVYMEL SSLRSEDTAVYYCAREGATGTWYFDVWG QGTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H2 L3 | VH | 83 | EVQLVQSGAEVKKPGASVKVSCKASGYT FTSYTMHWVRQAPGQGLEWMGDINPNY GGTSYAQKFQGRVTMTRDTSTSTVYMEL SSLRSEDTAVYYCAREGATGTWYFDVWG QGTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H2 L4 | VH | 83 | EVQLVQSGAEVKKPGASVKVSCKASGYT FTSYTMHWVRQAPGQGLEWMGDINPNY GGTSYAQKFQGRVTMTRDTSTSTVYMEL SSLRSEDTAVYYCAREGATGTWYFDVWG QGTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H2 L5 | VH | 83 | EVQLVQSGAEVKKPGASVKVSCKASGYT FTSYTMHWVRQAPGQGLEWMGDINPNY GGTSYAQKFQGRVTMTRDTSTSTVYMEL SSLRSEDTAVYYCAREGATGTWYFDVWG QGTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H2 L6 | VH | 83 | EVQLVQSGAEVKKPGASVKVSCKASGYT FTSYTMHWVRQAPGQGLEWMGDINPNY GGTSYAQKFQGRVTMTRDTSTSTVYMEL SSLRSEDTAVYYCAREGATGTWYFDVWG QGTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H3 L1 | VH | 84 | EVQLVQSGAEVKKPGASVKVSCKASGYT FTSYTMHWVRQAPGQGLEWMGDINPNY GGTSYAQKFQGRVTLTVDTSTSTVYMELS SLRSEDTAVYYCAREGATGTWYFDVWGQ GTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H3 L2 | VH | 84 | EVQLVQSGAEVKKPGASVKVSCKASGYT FTSYTMHWVRQAPGQGLEWMGDINPNY GGTSYAQKFQGRVTLTVDTSTSTVYMELS SLRSEDTAVYYCAREGATGTWYFDVWGQ GTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H3 L3 | VH | 84 | EVQLVQSGAEVKKPGASVKVSCKASGYT FTSYTMHWVRQAPGQGLEWMGDINPNY GGTSYAQKFQGRVTLTVDTSTSTVYMELS SLRSEDTAVYYCAREGATGTWYFDVWGQ GTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H3 L4 | VH | 84 | EVQLVQSGAEVKKPGASVKVSCKASGYT FTSYTMHWVRQAPGQGLEWMGDINPNY GGTSYAQKFQGRVTLTVDTSTSTVYMELS SLRSEDTAVYYCAREGATGTWYFDVWGQ GTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H3 L5 | VH | 84 | EVQLVQSGAEVKKPGASVKVSCKASGYT FTSYTMHWVRQAPGQGLEWMGDINPNY GGTSYAQKFQGRVTLTVDTSTSTVYMELS SLRSEDTAVYYCAREGATGTWYFDVWGQ GTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H3 L6 | VH | 84 | EVQLVQSGAEVKKPGASVKVSCKASGYT FTSYTMHWVRQAPGQGLEWMGDINPNY GGTSYAQKFQGRVTLTVDTSTSTVYMELS SLRSEDTAVYYCAREGATGTWYFDVWGQ GTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H4 L1 | VH | 85 | EVQLVQSGAEVKKPGASVKVSCKASGYT FTSYTMHWVRQAPGQGLEWMGDINPNY GGTSYNQKFQGRVTLTVDTSTSTVYMELS SLRSEDTAVYYCAREGATGTWYFDVWGQ GTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H4 L2 | VH | 85 | EVQLVQSGAEVKKPGASVKVSCKASGYT FTSYTMHWVRQAPGQGLEWMGDINPNY GGTSYNQKFQGRVTLTVDTSTSTVYMELS SLRSEDTAVYYCAREGATGTWYFDVWGQ GTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H4 L3 | VH | 85 | EVQLVQSGAEVKKPGASVKVSCKASGYT FTSYTMHWVRQAPGQGLEWMGDINPNY GGTSYNQKFQGRVTLTVDTSTSTVYMELS SLRSEDTAVYYCAREGATGTWYFDVWGQ GTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H4 L4 | VH | 85 | EVQLVQSGAEVKKPGASVKVSCKASGYT FTSYTMHWVRQAPGQGLEWMGDINPNY GGTSYNQKFQGRVTLTVDTSTSTVYMELS SLRSEDTAVYYCAREGATGTWYFDVWGQ GTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H4 L5 | VH | 85 | EVQLVQSGAEVKKPGASVKVSCKASGYT FTSYTMHWVRQAPGQGLEWMGDINPNY GGTSYNQKFQGRVTLTVDTSTSTVYMELS SLRSEDTAVYYCAREGATGTWYFDVWGQ GTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H4 L6 | VH | 85 | EVQLVQSGAEVKKPGASVKVSCKASGYT FTSYTMHWVRQAPGQGLEWMGDINPNY GGTSYNQKFQGRVTLTVDTSTSTVYMELS SLRSEDTAVYYCAREGATGTWYFDVWGQ GTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H5 L1 | VH | 86 | EVQLVQSGAEVKKPGASVKVSCKASGYT FTSYTMHWVRQAPGQGLEWMGDINPNY GGTSYNEKFKDRVTMTTDTSTSTAYMEL RSLRSDDTAVYYCAREGATGTWYFDVWG QGTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H5 L2 | VH | 86 | EVQLVQSGAEVKKPGASVKVSCKASGYT FTSYTMHWVRQAPGQGLEWMGDINPNY GGTSYNEKFKDRVTMTTDTSTSTAYMEL RSLRSDDTAVYYCAREGATGTWYFDVWG QGTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H5 L3 | VH | 86 | EVQLVQSGAEVKKPGASVKVSCKASGYT FTSYTMHWVRQAPGQGLEWMGDINPNY GGTSYNEKFKDRVTMTTDTSTSTAYMEL RSLRSDDTAVYYCAREGATGTWYFDVWG QGTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H5 L4 | VH | 86 | EVQLVQSGAEVKKPGASVKVSCKASGYT FTSYTMHWVRQAPGQGLEWMGDINPNY GGTSYNEKFKDRVTMTTDTSTSTAYMEL RSLRSDDTAVYYCAREGATGTWYFDVWG QGTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H5 L5 | VH | 86 | EVQLVQSGAEVKKPGASVKVSCKASGYT FTSYTMHWVRQAPGQGLEWMGDINPNY GGTSYNEKFKDRVTMTTDTSTSTAYMEL RSLRSDDTAVYYCAREGATGTWYFDVWG QGTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H5 L6 | VH | 86 | EVQLVQSGAEVKKPGASVKVSCKASGYT FTSYTMHWVRQAPGQGLEWMGDINPNY GGTSYNEKFKDRVTMTTDTSTSTAYMEL RSLRSDDTAVYYCAREGATGTWYFDVWG QGTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H6 L1 | VH | 87 | EVQLVQSGAEVKKPGASVKVSCKASGYT FTSYTMHWVRQAPGQGLEWMGDINPNY GGTSYNEKFKDRATMTTDTSTSTAYMEL RSLRSDDTAVYYCAREGATGTWYFDVWG QGTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H6 L2 | VH | 87 | EVQLVQSGAEVKKPGASVKVSCKASGYT FTSYTMHWVRQAPGQGLEWMGDINPNY GGTSYNEKFKDRATMTTDTSTSTAYMEL RSLRSDDTAVYYCAREGATGTWYFDVWG QGTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H6 L3 | VH | 87 | EVQLVQSGAEVKKPGASVKVSCKASGYT FTSYTMHWVRQAPGQGLEWMGDINPNY GGTSYNEKFKDRATMTTDTSTSTAYMEL RSLRSDDTAVYYCAREGATGTWYFDVWG QGTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H6 L4 | VH | 87 | EVQLVQSGAEVKKPGASVKVSCKASGYT FTSYTMHWVRQAPGQGLEWMGDINPNY GGTSYNEKFKDRATMTTDTSTSTAYMEL RSLRSDDTAVYYCAREGATGTWYFDVWG QGTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H6 L5 | VH | 87 | EVQLVQSGAEVKKPGASVKVSCKASGYT FTSYTMHWVRQAPGQGLEWMGDINPNY GGTSYNEKFKDRATMTTDTSTSTAYMEL RSLRSDDTAVYYCAREGATGTWYFDVWG QGTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H6 L6 | VH | 87 | EVQLVQSGAEVKKPGASVKVSCKASGYT FTSYTMHWVRQAPGQGLEWMGDINPNY GGTSYNEKFKDRATMTTDTSTSTAYMEL RSLRSDDTAVYYCAREGATGTWYFDVWG QGTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H7 L1 | VH | 88 | EVQLVQSGAEVKKPGASVKVSCKASGYT FTSYTMHWVRQAPGQGLEWIGDINPNYG GTSYNEKFKDRATLTTDTSTSTAYMELRS LRSDDTAVYYCAREGATGTWYFDVWGQ GTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H7 L2 | VH | 88 | EVQLVQSGAEVKKPGASVKVSCKASGYT FTSYTMHWVRQAPGQGLEWIGDINPNYG GTSYNEKFKDRATLTTDTSTSTAYMELRS LRSDDTAVYYCAREGATGTWYFDVWGQ GTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H7 L3 | VH | 88 | EVQLVQSGAEVKKPGASVKVSCKASGYT FTSYTMHWVRQAPGQGLEWIGDINPNYG GTSYNEKFKDRATLTTDTSTSTAYMELRS LRSDDTAVYYCAREGATGTWYFDVWGQ GTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H7 L4 | VH | 88 | EVQLVQSGAEVKKPGASVKVSCKASGYT FTSYTMHWVRQAPGQGLEWIGDINPNYG GTSYNEKFKDRATLTTDTSTSTAYMELRS LRSDDTAVYYCAREGATGTWYFDVWGQ GTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H7 L5 | VH | 88 | EVQLVQSGAEVKKPGASVKVSCKASGYT FTSYTMHWVRQAPGQGLEWIGDINPNYG GTSYNEKFKDRATLTTDTSTSTAYMELRS LRSDDTAVYYCAREGATGTWYFDVWGQ GTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H7 L6 | VH | 88 | EVQLVQSGAEVKKPGASVKVSCKASGYT FTSYTMHWVRQAPGQGLEWIGDINPNYG GTSYNEKFKDRATLTTDTSTSTAYMELRS LRSDDTAVYYCAREGATGTWYFDVWGQ GTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H8 L1 | VH | 89 | EVQLVQSGAEVKKPGASVKVSCKASGYT FTSYTMHWVRQAPGQGLEWIGDINPNYG GTSYNEKFKDRATLTTDTSTSTAYMELRS LRSDDTAVYYCAREGATGTWYFDVWGQ GTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H8 L2 | VH | 89 | EVQLVQSGAEVKKPGASVKVSCKASGYT FTSYTMHWVRQAPGQGLEWIGDINPNYG GTSYNEKFKDRATLTVDTSTSTAYMELRS LRSDDTAVYYCAREGATGTWYFDVWGQ GTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H8 L3 | VH | 89 | EVQLVQSGAEVKKPGASVKVSCKASGYT FTSYTMHWVRQAPGQGLEWIGDINPNYG GTSYNEKFKDRATLTVDTSTSTAYMELRS LRSDDTAVYYCAREGATGTWYFDVWGQ GTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H8 L4 | VH | 89 | EVQLVQSGAEVKKPGASVKVSCKASGYT FTSYTMHWVRQAPGQGLEWIGDINPNYG GTSYNEKFKDRATLTVDTSTSTAYMELRS LRSDDTAVYYCAREGATGTWYFDVWGQ GTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H8 L5 | VH | 89 | EVQLVQSGAEVKKPGASVKVSCKASGYT FTSYTMHWVRQAPGQGLEWIGDINPNYG GTSYNEKFKDRATLTVDTSTSTAYMELRS LRSDDTAVYYCAREGATGTWYFDVWGQ GTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H8 L6 | VH | 89 | EVQLVQSGAEVKKPGASVKVSCKASGYT FTSYTMHWVRQAPGQGLEWIGDINPNYG GTSYNEKFKDRATLTVDTSTSTAYMELRS LRSDDTAVYYCAREGATGTWYFDVWGQ GTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |
| hz103E9B3H9 L1 | VH | 90 | EVQLVQSGAEVKKPGASVKVSCKASGYT FTSYTMHWVRQAPGQGLEWIGDINPNYG GTSYNEKFKDRATLTVDKSTSTAYMELRS LRSDDTAVYYCAREGATGTWYFDVWGQ GTTVTVSS |
| | VL | 91 | DIQMTQSPSSLSASVGDRVTITCRASSSVS FLNWYQQKPGKAPKLLIYLTSSLQSGVPS RFSGSGSGTDFTLTISSLQPEDFATYYCQQ WRSNPPTFGGGTKVEIK |
| hz103E9B3H9 L2 | VH | 90 | EVQLVQSGAEVKKPGASVKVSCKASGYT FTSYTMHWVRQAPGQGLEWIGDINPNYG GTSYNEKFKDRATLTVDKSTSTAYMELRS LRSDDTAVYYCAREGATGTWYFDVWGQ GTTVTVSS |
| | VL | 92 | DIQMTQSPSSLSASVGDRVTITCRASSSVS FLFWYQQKPGKAPKLLIYLTSSLASGVPS RFSGSGSGTDFTLTISSLQPEDFATYYCQQ WRSNPPTFGGGTKVEIK |
| hz103E9B3H9 L3 | VH | 90 | EVQLVQSGAEVKKPGASVKVSCKASGYT FTSYTMHWVRQAPGQGLEWIGDINPNYG GTSYNEKFKDRATLTVDKSTSTAYMELRS LRSDDTAVYYCAREGATGTWYFDVWGQ GTTVTVSS |
| | VL | 93 | DIQMTQSPSSLSASVGDRVTITCRASSSVS FMFWYQQKPGKAPKPLIYLTSSLASGVPS RFSGSGSGTDFTLTISSLQPEDFATYYCQQ WRSNPPTFGGGTKVEIK |
| hz103E9B3H9 L4 | VH | 90 | EVQLVQSGAEVKKPGASVKVSCKASGYT FTSYTMHWVRQAPGQGLEWIGDINPNYG GTSYNEKFKDRATLTVDKSTSTAYMELRS LRSDDTAVYYCAREGATGTWYFDVWGQ GTTVTVSS |
| | VL | 94 | DIQMTQSPSSLSASVGDRVTITCSASSSVS FMFWYQQKPGKAPKLLIYLTSNLASGVPS RFSGSGSGTDFTLTISSLQPEDFATYYCQQ WRSNPPTFGGGTKVEIK |
| hz103E9B3H9 L5 | VH | 90 | EVQLVQSGAEVKKPGASVKVSCKASGYT FTSYTMHWVRQAPGQGLEWIGDINPNYG GTSYNEKFKDRATLTVDKSTSTAYMELRS LRSDDTAVYYCAREGATGTWYFDVWGQ GTTVTVSS |
| | VL | 95 | DIQMTQSPSSLSASVGDRVTITCSASSSVS FMFWYQQKPGKAPKPLIYLTSNLASGVPS RFSGSGSGTDFTLTISSLQPEDFATYYCQQ WRSNPPTFGGGTKVEIK |
| hz103E9B3H9 L6 | VH | 90 | EVQLVQSGAEVKKPGASVKVSCKASGYT FTSYTMHWVRQAPGQGLEWIGDINPNYG GTSYNEKFKDRATLTVDKSTSTAYMELRS LRSDDTAVYYCAREGATGTWYFDVWGQ GTTVTVSS |
| | VL | 96 | DIQMTQSPSSLSASVGDRVTITCSASSSVS FMFWYQQKPGKAPKPWIYLTSNLASGVP SRFSGSGSGTDFTLTISSLQPEDFATYYCQ QWRSNPPTFGGGTKVEIK |

## Claims

1. An antibody or antigen binding fragment thereof specifically binding to L1CAM comprising a light chain variable region comprising light chain complementarity determining regions LCDR1, LCDR2 and LCDR3 and/or a heavy chain variable region comprising heavy chain complementarity determining regions HCDR1, HCDR2 and HCDR3, wherein the antibody or antigen binding fragment thereof can specifically bind to the Ig1 or FN-4 domain of L1-CAM and does not have the same or equivalent combination of CDRs as antibody L1-9. 3 or UJ127.

2. The antibody or antigen binding fragment according to claim 1, wherein the heavy chain variable region that comprises HCDR1, HCDR2 and HCDR3 comprising at least 80%, 90% or 100% identity to the HCDR1, HCDR2 and HCDR3 of the heavy chain variable region sequences selected from the group comprising SEQ ID NOs: 43, 45, 47, 49 and 51, and the light chain variable region that comprises LCDR1, LCDR2 and LCDR3 comprising at least 80% identity to the LCDR1, LCDR2 and LCDR3 of the light chain variable region sequences selected from the group comprising SEQ ID NOs: 44, 46, 48, 50 and 52.

3. The antibody or antigen binding fragment according to claim 1 or 2, comprising a heavy chain variable region and a light chain variable region, wherein the heavy chain variable region and the light chain variable region comprise a combination of CDRs selected from the group comprising of:
(1) HCDR1, HCDR2 and HCDR3 comprising at least 80% identity to HCDR1, HCDR2 and HCDR3 of the heavy chain variable region sequence of SEQ ID NO: 43, LCDR1, LCDR2 and LCDR3 comprising at least 80% identity to LCDR1, LCDR2 and LCDR3 of the light chain variable region sequence of SEQ ID NO: 44;
(2) HCDR1, HCDR2 and HCDR3 comprising at least 80% identity to HCDR1, HCDR2 and HCDR3 of the heavy chain variable region sequence of SEQ ID NO: 45, LCDR1, LCDR2 and LCDR3 comprising at least 80% identity to LCDR1, LCDR2 and LCDR3 of the light chain variable region sequence of SEQ ID NO: 46;
(3) HCDR1, HCDR2 and HCDR3 comprising at least 80% identity to HCDR1, HCDR2 and HCDR3 of the heavy chain variable region sequence of SEQ ID NO: 47, LCDR1, LCDR2 and LCDR3 comprising at least 80% identity to LCDR1, LCDR2 and LCDR3 of the light chain variable region sequence of SEQ ID NO: 48;
(4) HCDR1, HCDR2 and HCDR3 comprising at least 80% identity to HCDR1, HCDR2 and HCDR3 of the heavy chain variable region sequence of SEQ ID NO: 49, LCDR1, LCDR2 and LCDR3 comprising at least 80% identity to LCDR1, LCDR2 and LCDR3 of the light chain variable region sequence of SEQ ID NO: 50; And
(5) HCDR1, HCDR2 and HCDR3 comprising at least 80% identity to HCDR1, HCDR2 and HCDR3 of the heavy chain variable region sequence of SEQ ID NO: 51, LCDR1, LCDR2 and LCDR3 comprising at least 80% identity to LCDR1, LCDR2 and LCDR3 of the light chain variable region sequence of SEQ ID NO: 52.

4. The antibody or antigen-binding fragment according to any one of claims 1 to 3, wherein:
(1) the HCDR1 comprises at least 80% identity to a sequence selected from SEQ ID NO: 1, SEQ ID NO: 7, SEQ ID NO: 13, SEQ ID NO: 19, SEQ ID NO: 25, SEQ ID NO: 31, SEQ ID NO: 37;
(2) the HCDR2 comprises at least 80% identity to a sequence selected from SEQ ID NO: 2, SEQ ID NO: 8, SEQ ID NO: 14, SEQ ID NO: 20, SEQ ID NO: 26, SEQ ID NO: 32, SEQ ID NO: 38;
(3) the HCDR3 comprises at least 80% identity to a sequence selected from SEQ ID NO: 3, SEQ ID NO: 9, SEQ ID NO: 15, SEQ ID NO: 21, SEQ ID NO: 27, SEQ ID NO: 33, SEQ ID NO: 39;
(4) the LCDR1 comprises at least 80% identity to a sequence selected from SEQ ID NO: 4, SEQ ID NO: 10, SEQ ID NO: 16, SEQ ID NO: 22, SEQ ID NO: 28, SEQ ID NO: 34, SEQ ID NO: 40;
(5) the LCDR2 comprises at least 80% identity to a sequence selected from SEQ ID NO: 5, SEQ ID NO: 11, SEQ ID NO: 17, SEQ ID NO: 23, SEQ ID NO: 29, SEQ ID NO: 35, SEQ ID NO: 41;
(6) the LCDR3 comprises at least 80% identity to a sequence selected from SEQ ID NO: 6, SEQ ID NO: 12, SEQ ID NO: 18, SEQ ID NO: 24, SEQ ID NO: 30, SEQ ID NO: 36, SEQ ID NO: 42.

5. The antibody or antigen-binding fragment according to any one of claims 1 to 4, wherein:
(1) the HCDR1 comprises the amino acid sequence set forth in SEQ ID NO: 1;
(2) the HCDR2 comprises the amino acid sequence set forth in SEQ ID NO: 2;
(3) the HCDR3 comprises the amino acid sequence set forth in SEQ ID NO: 3;
(4) the LCDR1 comprises the amino acid sequence set forth in SEQ ID NO: 4;
(5) the LCDR2 comprises the amino acid sequence set forth in SEQ ID NO: 5;
(6) the LCDR3 comprises the amino acid sequence set forth in SEQ ID NO: 6.

6. The antibody or antigen-binding fragment according to any one of claims 1 to 4, wherein:
(1) the HCDR1 comprises the amino acid sequence set forth in SEQ ID NO: 7;
(2) the HCDR2 comprises the amino acid sequence set forth in SEQ ID NO: 8;
(3) the HCDR3 comprises the amino acid sequence set forth in SEQ ID NO: 9;
(4) the LCDR1 comprises the amino acid sequence set forth in SEQ ID NO: 10;
(5) the LCDR2 comprises the amino acid sequence set forth in SEQ ID NO: 11;
(6) the LCDR3 comprises the amino acid sequence set forth in SEQ ID NO: 12.

7. The antibody or antigen-binding fragment according to any one of claims 1 to 4, wherein:
(1) the HCDR1 comprises the amino acid sequence set forth in SEQ ID NO: 13;
(2) the HCDR2 comprises the amino acid sequence set forth in SEQ ID NO: 14;
(3) the HCDR3 comprises the amino acid sequence set forth in SEQ ID NO: 15;
(4) the LCDR1 comprises the amino acid sequence set forth in SEQ ID NO: 16;
(5) the LCDR2 comprises the amino acid sequence set forth in SEQ ID NO: 17;
(6) the LCDR3 comprises the amino acid sequence set forth in SEQ ID NO: 18.

8. The antibody or antigen-binding fragment according to any one of claims 1 to 4, wherein:
(1) the HCDR1 comprises the amino acid sequence set forth in SEQ ID NO: 19;
(2) the HCDR2 comprises the amino acid sequence set forth in SEQ ID NO: 20;
(3) the HCDR3 comprises an amino acid sequence as set forth in SEQ ID NO: 21;
(4) the LCDR1 comprises the amino acid sequence set forth in SEQ ID NO: 22;
(5) the LCDR2 comprises the amino acid sequence set forth in SEQ ID NO: 23;
(6) the LCDR3 comprises the amino acid sequence set forth in SEQ ID NO: 24.

9. The antibody or antigen-binding fragment according to any one of claims 1 to 4, wherein:
(1) the HCDR1 comprises the amino acid sequence set forth in SEQ ID NO: 25;
(2) the HCDR2 comprises the amino acid sequence set forth in SEQ ID NO: 26;
(3) the HCDR3 comprises the amino acid sequence set forth in SEQ ID NO: 27;
(4) the LCDR1 comprises the amino acid sequence set forth in SEQ ID NO: 28;
(5) the LCDR2 comprises the amino acid sequence set forth in SEQ ID NO: 29;
(6) the LCDR3 comprises the amino acid sequence set forth in SEQ ID NO: 30.

10. The antibody or antigen-binding fragment according to any one of claims 1 to 4, wherein:
(1) the HCDR1 comprises the amino acid sequence set forth in SEQ ID NO: 31;
(2) the HCDR2 comprises the amino acid sequence set forth in SEQ ID NO: 32;
(3) the HCDR3 comprises the amino acid sequence set forth in SEQ ID NO: 33;
(4) the LCDR1 comprises the amino acid sequence set forth in SEQ ID NO: 34;
(5) the LCDR2 comprises the amino acid sequence set forth in SEQ ID NO: 35;
(6) the LCDR3 comprises the amino acid sequence set forth in SEQ ID NO: 36.

11. The antibody or antigen-binding fragment according to any one of claims 1 to 4, wherein:
(1) the HCDR1 comprises the amino acid sequence set forth in SEQ ID NO: 37;
(2) the HCDR2 comprises the amino acid sequence set forth in SEQ ID NO: 38;
(3) the HCDR3 comprises the amino acid sequence set forth in SEQ ID NO: 39;
(4) the LCDR1 comprises the amino acid sequence set forth in SEQ ID NO: 40;
(5) the LCDR2 comprises the amino acid sequence set forth in SEQ ID NO: 41;
(6) the LCDR3 comprises the amino acid sequence set forth in SEQ ID NO: 42.

12. The antibody or antigen-binding fragment of any one of claims 1 to 11, wherein the heavy chain variable region comprises an amino acid sequence comprising at least 90%, at least 95%, or 100% sequence identity to the amino acid sequence set forth in SEQ ID NO: 43 or to SEQ ID NO: 43.

13. The antibody or antigen-binding fragment of any one of claims 1 to 11, wherein the light chain variable region comprises the amino acid sequence set forth in SEQ ID NO: 44 or an amino acid sequence comprising at least 90%, at least 95%, or 100% sequence identity to SEQ ID NO: 44.

14. The antibody or antigen-binding fragment of any one of claims 1 to 11, wherein the heavy chain variable region comprises the amino acid sequence set forth in SEQ ID NO: 45 or an amino acid sequence comprising at least 90%, at least 95% or more sequence identity to SEQ ID NO: 45.

15. The antibody or antigen-binding fragment of any one of claims 1 to 11, wherein the light chain variable region comprises the amino acid sequence set forth in SEQ ID NO: 46 or an amino acid sequence comprising at least 90%, at least 95% or more sequence identity to SEQ ID NO: 46.

16. The antibody or antigen-binding fragment of any one of claims 1 to 11, wherein the heavy chain variable region comprises the amino acid sequence set forth in SEQ ID NO: 47 or an amino acid sequence comprising at least 90%, at least 95% or more sequence identity to SEQ ID NO: 47.

17. The antibody or antigen-binding fragment of any one of claims 1 to 11, wherein the light chain variable region comprises the amino acid sequence set forth in SEQ ID NO: 48 or an amino acid sequence comprising at least 90%, at least 95% or more sequence identity to SEQ ID NO: 48.

18. The antibody or antigen-binding fragment of any one of claims 1 to 9, wherein the heavy chain variable region comprises the amino acid sequence set forth in SEQ ID NO: 49 or an amino acid sequence comprising at least 90%, at least 95% or more sequence identity to SEQ ID NO: 49.

19. The antibody or antigen-binding fragment according to any one of claims 1 to 11, wherein the light chain variable region comprises the amino acid sequence set forth in SEQ ID NO: 50 or an amino acid sequence comprising at least 90%, at least 95% or more sequence identity to SEQ ID NO: 50.

20. The antibody or antigen-binding fragment according to any one of claims 1 to 11, wherein the heavy chain variable region comprises the amino acid sequence set forth in SEQ ID NO: 51 or an amino acid sequence comprising at least 90%, at least 95% or more sequence identity to SEQ ID NO: 51.

21. The antibody or antigen-binding fragment according to any one of claims 1 to 11, wherein the light chain variable region comprises the amino acid sequence set forth in SEQ ID NO: 52 or an amino acid sequence comprising at least 90%, at least 95% or more sequence identity to SEQ ID NO: 52.

22. The antibody or antigen-binding fragment according to any one of claims 1-21, wherein the antibody is a humanized antibody.

23. The antibody or antigen-binding fragment according to claim 22, comprising a heavy chain variable region and/or a light chain variable region, wherein the heavy chain variable region comprises one of the amino acid sequences set forth in SEQ ID NO: 53 or SEQ ID NO: 54 or SEQ ID NO: 55 or SEQ ID NO: 56, and the light chain variable region comprises one of the amino acid sequences set forth in SEQ ID NO: 57 or SEQ ID NO: 58 or SEQ ID NO: 59.

24. The antibody or antigen-binding fragment according to claim 23, which comprises a heavy chain variable region and/or a light chain variable region, wherein:
1) the heavy chain variable region comprises the amino acid sequence set forth in SEQ ID NO: 53, and the light chain variable region comprises the amino acid sequence set forth in SEQ ID NO: 57; or
2) the heavy chain variable region comprises the amino acid sequence set forth in SEQ ID NO: 53, and the light chain variable region comprises the amino acid sequence set forth in SEQ ID NO: 58; or
3) the heavy chain variable region comprises the amino acid sequence set forth in SEQ ID NO: 53, and the light chain variable region comprises the amino acid sequence set forth in SEQ ID NO: 59; or
4) the heavy chain variable region comprises the amino acid sequence set forth in SEQ ID NO: 54, and the light chain variable region comprises the amino acid sequence set forth in SEQ ID NO: 57; or
5) the heavy chain variable region comprises the amino acid sequence set forth in SEQ ID NO: 54, and the light chain variable region comprises the amino acid sequence set forth in SEQ ID NO: 58; or
6) the heavy chain variable region comprises the amino acid sequence set forth in SEQ ID NO: 54, and the light chain variable region comprises the amino acid sequence set forth in SEQ ID NO: 59; or
7) the heavy chain variable region comprises the amino acid sequence set forth in SEQ ID NO: 55, and the light chain variable region comprises the amino acid sequence set forth in SEQ ID NO: 57; or
8) the heavy chain variable region comprises the amino acid sequence set forth in SEQ ID NO: 55, and the light chain variable region comprises the amino acid sequence set forth in SEQ ID NO: 58; or
9) the heavy chain variable region comprises the amino acid sequence set forth in SEQ ID NO: 55, and the light chain variable region comprises the amino acid sequence set forth in SEQ ID NO: 59; or
10) the heavy chain variable region comprises the amino acid sequence set forth in SEQ ID NO: 56, and the light chain variable region comprises the amino acid sequence set forth in SEQ ID NO: 57; or
11) the heavy chain variable region comprises the amino acid sequence set forth in SEQ ID NO: 56, and the light chain variable region comprises the amino acid sequence set forth in SEQ ID NO: 58; or
12) the heavy chain variable region comprises the amino acid sequence set forth in SEQ ID NO: 56, and the light chain variable region comprises the amino acid sequence set forth in SEQ ID NO: 59.

25. The antibody or antigen-binding fragment according to claim 23, wherein the heavy chain variable region comprises one of the amino acid sequences set forth in SEQ ID NO: 60 or SEQ ID NO: 61 or SEQ ID NO: 62 or SEQ ID NO: 63, and the light chain variable region comprises one of the amino acid sequences set forth in SEQ ID NO: 64 or SEQ ID NO: 65 or SEQ ID NO: 66.

26. The antibody or antigen binding fragment according to claim 25, comprising a heavy chain variable region and/or a light chain variable region, wherein,
1) the heavy chain variable region comprises the amino acid sequence set forth in SEQ ID NO: 60, and the light chain variable region comprises the amino acid sequence set forth in SEQ ID NO: 64; or
2) the heavy chain variable region comprises the amino acid sequence set forth in SEQ ID NO: 60, and the light chain variable region comprises the amino acid sequence set forth in SEQ ID NO: 65; or
3) the heavy chain variable region comprises the amino acid sequence set forth in SEQ ID NO: 60, and the light chain variable region comprises the amino acid sequence set forth in SEQ ID NO: 66; or
4) the heavy chain variable region comprises the amino acid sequence set forth in SEQ ID NO: 61, and the light chain variable region comprises the amino acid sequence set forth in SEQ ID NO: 64; or
5) the heavy chain variable region comprises the amino acid sequence set forth in SEQ ID NO: 61, and the light chain variable region comprises the amino acid sequence set forth in SEQ ID NO: 65; or
6) the heavy chain variable region comprises the amino acid sequence set forth in SEQ ID NO: 61, and the light chain variable region comprises the amino acid sequence set forth in SEQ ID NO: 66; or
7) the heavy chain variable region comprises the amino acid sequence set forth in SEQ ID NO: 62, and the light chain variable region comprises the amino acid sequence set forth in SEQ ID NO: 64; or
8) the heavy chain variable region comprises the amino acid sequence set forth in SEQ ID NO: 62, and the light chain variable region comprises the amino acid sequence set forth in SEQ ID NO: 65; or
9) the heavy chain variable region comprises the amino acid sequence set forth in SEQ ID NO: 62, and the light chain variable region comprises the amino acid sequence set forth in SEQ ID NO: 66; or
10) the heavy chain variable region comprises the amino acid sequence set forth in SEQ ID NO: 63, and the light chain variable region comprises the amino acid sequence set forth in SEQ ID NO: 64; or
11) the heavy chain variable region comprises the amino acid sequence set forth in SEQ ID NO: 63, and the light chain variable region comprises the amino acid sequence set forth in SEQ ID NO: 65; or
12) the heavy chain variable region comprises the amino acid sequence set forth in SEQ ID NO: 63, and the light chain variable region comprises the amino acid sequence set forth in SEQ ID NO: 66.

27. The antibody or antigen-binding fragment according to claim 22, wherein the heavy chain variable region comprises one of the amino acid sequences set forth in SEQ ID NO: 67 or SEQ ID NO: 68 or SEQ ID NO: 69 or SEQ ID NO: 70 or SEQ ID NO: 71 or SEQ ID NO: 72 or SEQ ID NO: 73 or SEQ ID NO: 74 and the light chain variable region comprises one of the amino acid sequences set forth in SEQ ID NO: 75 or SEQ ID NO: 76 or SEQ ID NO: 77 or SEQ ID NO: 78 or SEQ ID NO: 79 or SEQ ID NO: 80 or SEQ ID NO: 81.

28. The antibody or antigen binding fragment according to claim 27, comprising a heavy chain variable region and/or a light chain variable region, wherein,
1) the heavy chain variable region comprises the amino acid sequence set forth in SEQ ID NO: 67, and the light chain variable region comprises the amino acid sequence set forth in SEQ ID NO: 75; Or
2) the heavy chain variable region comprises the amino acid sequence set forth in SEQ ID NO: 67, and the light chain variable region comprises the amino acid sequence set forth in SEQ ID NO: 76; Or
3) the heavy chain variable region comprises the amino acid sequence set forth in SEQ ID NO: 67, and the light chain variable region comprises the amino acid sequence set forth in SEQ ID NO: 77; Or
4) the heavy chain variable region comprises the amino acid sequence set forth in SEQ ID NO: 67, and the light chain variable region comprises the amino acid sequence set forth in SEQ ID NO: 78; Or
5) the heavy chain variable region comprises the amino acid sequence set forth in SEQ ID NO: 67 and the light chain variable region comprises the amino acid sequence set forth in SEQ ID NO: 79; Or
6) the heavy chain variable region comprises the amino acid sequence set forth in SEQ ID NO: 67, and the light chain variable region comprises the amino acid sequence set forth in SEQ ID NO: 80; Or
7) the heavy chain variable region comprises the amino acid sequence set forth in SEQ ID NO: 67, and the light chain variable region comprises the amino acid sequence set forth in SEQ ID NO: 81; Or
8) the heavy chain variable region comprises the amino acid sequence set forth in SEQ ID NO: 68 and the light chain variable region comprises the amino acid sequence set forth in SEQ ID NO: 75; Or
9) the heavy chain variable region comprises the amino acid sequence set forth in SEQ ID NO: 68, and the light chain variable region comprises the amino acid sequence set forth in SEQ ID NO: 76; Or
10) the heavy chain variable region comprises the amino acid sequence set forth in SEQ ID NO: 68, and the light chain variable region comprises the amino acid sequence set forth in SEQ ID NO: 77; Or
11) the heavy chain variable region comprises the amino acid sequence set forth in SEQ ID NO: 68, and the light chain variable region comprises the amino acid sequence set forth in SEQ ID NO: 78; Or
12) the heavy chain variable region comprises the amino acid sequence set forth in SEQ ID NO: 68, and the light chain variable region comprises the amino acid sequence set forth in SEQ ID NO: 79; Or
13) the heavy chain variable region comprises the amino acid sequence set forth in SEQ ID NO: 68, and the light chain variable region comprises the amino acid sequence set forth in SEQ ID NO: 80; Or
14) the heavy chain variable region comprises the amino acid sequence set forth in SEQ ID NO: 68, and the light chain variable region comprises the amino acid sequence set forth in SEQ ID NO: 81; Or
15) the heavy chain variable region comprises the amino acid sequence set forth in SEQ ID NO: 69, and the light chain variable region comprises the amino acid sequence set forth in SEQ ID NO: 75; Or
16) the heavy chain variable region comprises the amino acid sequence set forth in SEQ ID NO: 69, and the light chain variable region comprises the amino acid sequence set forth in SEQ ID NO: 76; Or
17) the heavy chain variable region comprises the amino acid sequence set forth in SEQ ID NO: 69, and the light chain variable region comprises the amino acid sequence set forth in SEQ ID NO: 77; Or
18) the heavy chain variable region comprises the amino acid sequence set forth in SEQ ID NO: 69, and the light chain variable region comprises the amino acid sequence set forth in SEQ ID NO: 78; Or
19) the heavy chain variable region comprises the amino acid sequence set forth in SEQ ID NO: 69, and the light chain variable region comprises the amino acid sequence set forth in SEQ ID NO: 79; Or
20) the heavy chain variable region comprises the amino acid sequence set forth in SEQ ID NO: 69, and the light chain variable region comprises the amino acid sequence set forth in SEQ ID NO: 80; Or
21) the heavy chain variable region comprises the amino acid sequence set forth in SEQ ID NO: 69, and the light chain variable region comprises the amino acid sequence set forth in SEQ ID NO: 81; Or
22) the heavy chain variable region comprises the amino acid sequence set forth in SEQ ID NO: 70, and the light chain variable region comprises the amino acid sequence set forth in SEQ ID NO: 75; Or
23) the heavy chain variable region comprises the amino acid sequence set forth in SEQ ID NO: 70, and the light chain variable region comprises the amino acid sequence set forth in SEQ ID NO: 76; Or
24) the heavy chain variable region comprises the amino acid sequence set forth in SEQ ID NO: 70, and the light chain variable region comprises the amino acid sequence set forth in SEQ ID NO: 77; Or
25) the heavy chain variable region comprises the amino acid sequence set forth in SEQ ID NO: 70, and the light chain variable region comprises the amino acid sequence set forth in SEQ ID NO: 78; Or
26) the heavy chain variable region comprises the amino acid sequence set forth in SEQ ID NO: 70, and the light chain variable region comprises the amino acid sequence set forth in SEQ ID NO: 79; Or
27) the heavy chain variable region comprises the amino acid sequence set forth in SEQ ID NO: 70, and the light chain variable region comprises the amino acid sequence set forth in SEQ ID NO: 80; Or
28) the heavy chain variable region comprises the amino acid sequence set forth in SEQ ID NO: 70, and the light chain variable region comprises the amino acid sequence set forth in SEQ ID NO: 81; Or
29) the heavy chain variable region comprises the amino acid sequence set forth in SEQ ID NO: 71, and the light chain variable region comprises the amino acid sequence set forth in SEQ ID NO: 75; Or
30) the heavy chain variable region comprises the amino acid sequence set forth in SEQ ID NO: 71, and the light chain variable region comprises the amino acid sequence set forth in SEQ ID NO: 76; Or
31) the heavy chain variable region comprises the amino acid sequence set forth in SEQ ID NO: 71, and the light chain variable region comprises the amino acid sequence set forth in SEQ ID NO: 77; Or
32) the heavy chain variable region comprises the amino acid sequence set forth in SEQ ID NO: 71, and the light chain variable region comprises the amino acid sequence set forth in SEQ ID NO: 78; Or
33) the heavy chain variable region comprises the amino acid sequence set forth in SEQ ID NO: 71, and the light chain variable region comprises the amino acid sequence set forth in SEQ ID NO: 79; Or
34) the heavy chain variable region comprises the amino acid sequence set forth in SEQ ID NO: 71, and the light chain variable region comprises the amino acid sequence set forth in SEQ ID NO: 80; Or
35) the heavy chain variable region comprises the amino acid sequence set forth in SEQ ID NO: 71, and the light chain variable region comprises the amino acid sequence set forth in SEQ ID NO: 81; Or
36) the heavy chain variable region comprises the amino acid sequence set forth in SEQ ID NO: 72, and the light chain variable region comprises the amino acid sequence set forth in SEQ ID NO: 75; Or
37) the heavy chain variable region comprises the amino acid sequence set forth in SEQ ID NO: 72, and the light chain variable region comprises the amino acid sequence set forth in SEQ ID NO: 76; Or
38) the heavy chain variable region comprises the amino acid sequence set forth in SEQ ID NO: 72 and the light chain variable region comprises the amino acid sequence set forth in SEQ ID NO: 77; Or
39) the heavy chain variable region comprises the amino acid sequence set forth in SEQ ID NO: 72, and the light chain variable region comprises the amino acid sequence set forth in SEQ ID NO: 78; Or
40) the heavy chain variable region comprises the amino acid sequence set forth in SEQ ID NO: 72, and the light chain variable region comprises the amino acid sequence set forth in SEQ ID NO: 79; Or
41) the heavy chain variable region comprises the amino acid sequence set forth in SEQ ID NO: 72, and the light chain variable region comprises the amino acid sequence set forth in SEQ ID NO: 80; Or
42) the heavy chain variable region comprises the amino acid sequence set forth in SEQ ID NO: 72, and the light chain variable region comprises the amino acid sequence set forth in SEQ ID NO: 81; Or
43) the heavy chain variable region comprises the amino acid sequence set forth in SEQ ID NO: 73, and the light chain variable region comprises the amino acid sequence set forth in SEQ ID NO: 75; Or
44) the heavy chain variable region comprises the amino acid sequence set forth in SEQ ID NO: 73, and the light chain variable region comprises the amino acid sequence set forth in SEQ ID NO: 76; Or
45) the heavy chain variable region comprises the amino acid sequence set forth in SEQ ID NO: 73, and the light chain variable region comprises the amino acid sequence set forth in SEQ ID NO: 77; Or
46) the heavy chain variable region comprises the amino acid sequence set forth in SEQ ID NO: 73, and the light chain variable region comprises the amino acid sequence set forth in SEQ ID NO: 78; Or
47) the heavy chain variable region comprises the amino acid sequence set forth in SEQ ID NO: 73, and the light chain variable region comprises the amino acid sequence set forth in SEQ ID NO: 79; Or
48) the heavy chain variable region comprises the amino acid sequence set forth in SEQ ID NO: 73, and the light chain variable region comprises the amino acid sequence set forth in SEQ ID NO: 80; Or
49) the heavy chain variable region comprises the amino acid sequence set forth in SEQ ID NO: 73, and the light chain variable region comprises the amino acid sequence set forth in SEQ ID NO: 81; Or
50) the heavy chain variable region comprises the amino acid sequence set forth in SEQ ID NO: 74, and the light chain variable region comprises the amino acid sequence set forth in SEQ ID NO: 75; Or
51) the heavy chain variable region comprises the amino acid sequence set forth in SEQ ID NO: 74, and the light chain variable region comprises the amino acid sequence set forth in SEQ ID NO: 76; Or
52) the heavy chain variable region comprises the amino acid sequence set forth in SEQ ID NO: 74, and the light chain variable region comprises the amino acid sequence set forth in SEQ ID NO: 77; Or
53) the heavy chain variable region comprises the amino acid sequence set forth in SEQ ID NO: 74, and the light chain variable region comprises the amino acid sequence set forth in SEQ ID NO: 78; Or
54) the heavy chain variable region comprises the amino acid sequence set forth in SEQ ID NO: 74, and the light chain variable region comprises the amino acid sequence set forth in SEQ ID NO: 79; Or
55) the heavy chain variable region comprises the amino acid sequence set forth in SEQ ID NO: 74, and the light chain variable region comprises the amino acid sequence set forth in SEQ ID NO: 80; Or
56) the heavy chain variable region comprises the amino acid sequence set forth in SEQ ID NO: 74, and the light chain variable region comprises the amino acid sequence set forth in SEQ ID NO: 81.

29. The antibody or antigen-binding fragment according to claim 22, wherein the heavy chain variable region comprises one of the amino acid sequences set forth in SEQ ID NO: 82 or SEQ ID NO: 83 or SEQ ID NO: 84 or SEQ ID NO: 85 or SEQ ID NO: 86 or SEQ ID NO: 87 or SEQ ID NO: 88 or SEQ ID NO: 89 or SEQ ID NO: 90, and the light chain variable region comprises one of the amino acid sequences set forth in SEQ ID NO: 91 or SEQ ID NO: 92 or SEQ ID NO: 93 or SEQ ID NO: 94 or SEQ ID NO: 95 or SEQ ID NO: 96.

30. The antibody or antigen-binding fragment according to claim 29, which comprises a heavy chain variable region and/or a light chain variable region, wherein,
1) the heavy chain variable region comprises the amino acid sequence set forth in SEQ ID NO: 82, and the light chain variable region comprises the amino acid sequence set forth in SEQ ID NO: 91; Or
2) the heavy chain variable region comprises the amino acid sequence set forth in SEQ ID NO: 82, and the light chain variable region comprises the amino acid sequence set forth in SEQ ID NO: 92; Or
3) the heavy chain variable region comprises the amino acid sequence set forth in SEQ ID NO: 82, and the light chain variable region comprises the amino acid sequence set forth in SEQ ID NO: 93; Or
4) the heavy chain variable region comprises the amino acid sequence set forth in SEQ ID NO: 82, and the light chain variable region comprises the amino acid sequence set forth in SEQ ID NO: 94; Or
5) the heavy chain variable region comprises the amino acid sequence set forth in SEQ ID NO: 82, and the light chain variable region comprises the amino acid sequence set forth in SEQ ID NO: 95; Or
6) the heavy chain variable region comprises the amino acid sequence set forth in SEQ ID NO: 82, and the light chain variable region comprises the amino acid sequence set forth in SEQ ID NO: 96; Or
7) the heavy chain variable region comprises the amino acid sequence set forth in SEQ ID NO: 83, and the light chain variable region comprises the amino acid sequence set forth in SEQ ID NO: 91; Or
8) the heavy chain variable region comprises the amino acid sequence set forth in SEQ ID NO: 83, and the light chain variable region comprises the amino acid sequence set forth in SEQ ID NO: 92; Or
9) the heavy chain variable region comprises the amino acid sequence set forth in SEQ ID NO: 83, and the light chain variable region comprises the amino acid sequence set forth in SEQ ID NO: 93; Or
10) the heavy chain variable region comprises the amino acid sequence set forth in SEQ ID NO: 83, and the light chain variable region comprises the amino acid sequence set forth in SEQ ID NO: 94; Or
11) the heavy chain variable region comprises the amino acid sequence set forth in SEQ ID NO: 83, and the light chain variable region comprises the amino acid sequence set forth in SEQ ID NO: 95; Or
12) the heavy chain variable region comprises the amino acid sequence set forth in SEQ ID NO: 83, and the light chain variable region comprises the amino acid sequence set forth in SEQ ID NO: 96; Or
13) the heavy chain variable region comprises the amino acid sequence set forth in SEQ ID NO: 84, and the light chain variable region comprises the amino acid sequence set forth in SEQ ID NO: 91; Or
14) the heavy chain variable region comprises the amino acid sequence set forth in SEQ ID NO: 84, and the light chain variable region comprises the amino acid sequence set forth in SEQ ID NO: 92; Or
15) the heavy chain variable region comprises the amino acid sequence set forth in SEQ ID NO: 84, and the light chain variable region comprises the amino acid sequence set forth in SEQ ID NO: 93; Or
16) the heavy chain variable region comprises the amino acid sequence set forth in SEQ ID NO: 84, and the light chain variable region comprises the amino acid sequence set forth in SEQ ID NO: 94; Or
17) the heavy chain variable region comprises the amino acid sequence set forth in SEQ ID NO: 84, and the light chain variable region comprises the amino acid sequence set forth in SEQ ID NO: 95; Or
18) the heavy chain variable region comprises the amino acid sequence set forth in SEQ ID NO: 84, and the light chain variable region comprises the amino acid sequence set forth in SEQ ID NO: 96; Or
19) the heavy chain variable region comprises the amino acid sequence set forth in SEQ ID NO: 85, and the light chain variable region comprises the amino acid sequence set forth in SEQ ID NO: 91; Or
20) the heavy chain variable region comprises the amino acid sequence set forth in SEQ ID NO: 85, and the light chain variable region comprises the amino acid sequence set forth in SEQ ID NO: 92; Or
21) the heavy chain variable region comprises the amino acid sequence set forth in SEQ ID NO: 85, and the light chain variable region comprises the amino acid sequence set forth in SEQ ID NO: 93; Or
22) the heavy chain variable region comprises the amino acid sequence set forth in SEQ ID NO: 85, and the light chain variable region comprises the amino acid sequence set forth in SEQ ID NO: 94; Or
23) the heavy chain variable region comprises the amino acid sequence set forth in SEQ ID NO: 85, and the light chain variable region comprises the amino acid sequence set forth in SEQ ID NO: 95; Or
24) the heavy chain variable region comprises the amino acid sequence set forth in SEQ ID NO: 85, and the light chain variable region comprises the amino acid sequence set forth in SEQ ID NO: 96; Or
25) the heavy chain variable region comprises the amino acid sequence set forth in SEQ ID NO: 86, and the light chain variable region comprises the amino acid sequence set forth in SEQ ID NO: 91; Or
26) the heavy chain variable region comprises the amino acid sequence set forth in SEQ ID NO: 86, and the light chain variable region comprises the amino acid sequence set forth in SEQ ID NO: 92; Or
27) the heavy chain variable region comprises the amino acid sequence set forth in SEQ ID NO: 86, and the light chain variable region comprises the amino acid sequence set forth in SEQ ID NO: 93; Or
28) the heavy chain variable region comprises the amino acid sequence set forth in SEQ ID NO: 86, and the light chain variable region comprises the amino acid sequence set forth in SEQ ID NO: 94; Or
29) the heavy chain variable region comprises the amino acid sequence set forth in SEQ ID NO: 86, and the light chain variable region comprises the amino acid sequence set forth in SEQ ID NO: 95; Or
30) the heavy chain variable region comprises the amino acid sequence set forth in SEQ ID NO: 86, and the light chain variable region comprises the amino acid sequence set forth in SEQ ID NO: 96; Or
31) the heavy chain variable region comprises the amino acid sequence set forth in SEQ ID NO: 87, and the light chain variable region comprises the amino acid sequence set forth in SEQ ID NO: 91; Or
32) the heavy chain variable region comprises the amino acid sequence set forth in SEQ ID NO: 87, and the light chain variable region comprises the amino acid sequence set forth in SEQ ID NO: 92; Or
33) the heavy chain variable region comprises the amino acid sequence set forth in SEQ ID NO: 87, and the light chain variable region comprises the amino acid sequence set forth in SEQ ID NO: 93; Or
34) the heavy chain variable region comprises the amino acid sequence set forth in SEQ ID NO: 87, and the light chain variable region comprises the amino acid sequence set forth in SEQ ID NO: 94; Or
35) the heavy chain variable region comprises the amino acid sequence set forth in SEQ ID NO: 87, and the light chain variable region comprises the amino acid sequence set forth in SEQ ID NO: 95; Or
36) the heavy chain variable region comprises the amino acid sequence set forth in SEQ ID NO: 87, and the light chain variable region comprises the amino acid sequence set forth in SEQ ID NO: 96; Or
37) the heavy chain variable region comprises the amino acid sequence set forth in SEQ ID NO: 88, and the light chain variable region comprises the amino acid sequence set forth in SEQ ID NO: 91; Or
38) the heavy chain variable region comprises the amino acid sequence set forth in SEQ ID NO: 88, and the light chain variable region comprises the amino acid sequence set forth in SEQ ID NO: 92; Or
39) the heavy chain variable region comprises the amino acid sequence set forth in SEQ ID NO: 88, and the light chain variable region comprises the amino acid sequence set forth in SEQ ID NO: 93; Or
40) the heavy chain variable region comprises the amino acid sequence set forth in SEQ ID NO: 88, and the light chain variable region comprises the amino acid sequence set forth in SEQ ID NO: 94; Or
41) the heavy chain variable region comprises the amino acid sequence set forth in SEQ ID NO: 88, and the light chain variable region comprises the amino acid sequence set forth in SEQ ID NO: 95; Or
42) the heavy chain variable region comprises the amino acid sequence set forth in SEQ ID NO: 88, and the light chain variable region comprises the amino acid sequence set forth in SEQ ID NO: 96; Or
43) the heavy chain variable region comprises the amino acid sequence set forth in SEQ ID NO: 89, and the light chain variable region comprises the amino acid sequence set forth in SEQ ID NO: 91; Or
44) the heavy chain variable region comprises the amino acid sequence set forth in SEQ ID NO: 89, and the light chain variable region comprises the amino acid sequence set forth in SEQ ID NO: 92; Or
45) the heavy chain variable region comprises the amino acid sequence set forth in SEQ ID NO: 89, and the light chain variable region comprises the amino acid sequence set forth in SEQ ID NO: 93; Or
46) the heavy chain variable region comprises the amino acid sequence set forth in SEQ ID NO: 89, and the light chain variable region comprises the amino acid sequence set forth in SEQ ID NO: 94; Or
47) the heavy chain variable region comprises the amino acid sequence set forth in SEQ ID NO: 89, and the light chain variable region comprises the amino acid sequence set forth in SEQ ID NO: 95; Or
48) the heavy chain variable region comprises the amino acid sequence set forth in SEQ ID NO: 89, and the light chain variable region comprises the amino acid sequence set forth in SEQ ID NO: 96; Or
49) the heavy chain variable region comprises the amino acid sequence set forth in SEQ ID NO: 90, and the light chain variable region comprises the amino acid sequence set forth in SEQ ID NO: 91; Or
50) the heavy chain variable region comprises the amino acid sequence set forth in SEQ ID NO: 90, and the light chain variable region comprises the amino acid sequence set forth in SEQ ID NO: 92; Or
51) the heavy chain variable region comprises the amino acid sequence set forth in SEQ ID NO: 90, and the light chain variable region comprises the amino acid sequence set forth in SEQ ID NO: 93; Or
52) the heavy chain variable region comprises the amino acid sequence set forth in SEQ ID NO: 90, and the light chain variable region comprises the amino acid sequence set forth in SEQ ID NO: 94; Or
53) the heavy chain variable region comprises the amino acid sequence set forth in SEQ ID NO: 90, and the light chain variable region comprises the amino acid sequence set forth in SEQ ID NO: 95; Or
54) the heavy chain variable region comprises the amino acid sequence set forth in SEQ ID NO: 90, and the light chain variable region comprises the amino acid sequence set forth in SEQ ID NO: 96.

31. The antibody or antigen-binding fragment according to any one of claims 1-30, further comprising an Fc region, optionally that of a human immunoglobulin (Ig), or optionally that of a human IgG, whereas the Fc region is derived from human IgG1, IgG2, IgG3, IgG4, IgA1, IgA2 or IgM.

32. The antibody or antigen-binding fragment according to any one of claims 1-31, wherein the antigen-binding fragment is a scFv, scFv-Fc, Fv, Fab, Fab ', F (ab)₂ or F (ab ')₂

33. The antibody or antigen-binding fragment according to any one of claims 1-32 which is a monoclonal antibody, a bispecific antibody, a multi-specific antibody, a recombinant antibody, a chimeric antibody, a divalent antibody, an anti-idiotypic antibody, or a fusion protein.

34. An antibody-drug conjugate comprising the antibody or antigen-binding fragment according to any one of claims 1-33, and an additional effector molecule conjugate

35. A nucleic acid molecule encoding the antibody or antigen-binding fragment of any one of claims 1-33.

36. The nucleic acid molecule according to claim 39, operably linked to a promoter.

37. A recombinant expression vector comprising the nucleic acid molecule of claim 39 or 40.

38. A plasmid comprising the nucleic acid molecule of claim 39 or 40.

39. A host cell comprising the nucleic acid molecule of claim 39 or 40, the recombinant expression vector of claim 41 or the plasmid of claim 42.

40. A method for detecting the expression of L1CAM in a biological sample comprising the steps of contacting said biological sample with the antibody or antigen-binding fragment thereof of any one of claims 1-33 and detecting the presence or absence of an immune complex, wherein the presence of an immune complex indicates the presence of L1CAM in the biological sample

41. A pharmaceutical composition comprising, as an active substance, the antibody or the antigen-binding fragment thereof of any one of claims 1 to 33, the antibody-drug conjugate of any one of claims 34 to 38, and a pharmaceutically acceptable carrier

42. A kit comprising the antibody or antigen-binding fragment thereof according to any one of claims 1 to 33, the antibody-drug conjugate according to claim 34 and/or the pharmaceutical composition according to claim 45

43. A method of treating cancer comprising administering to a subject in need thereof the antibody or antigen-binding fragment thereof of any one of claims 1-33, the antibody-drug conjugate of claim 34, or the pharmaceutical composition of claim 45

44. The method according to claim 47, further comprising detecting the presence of L1-CAM in a cancer sample prior to administration of the medicament.
